(19) Europäisches Patentamt
European Patent Office
Office européen des brevets

(11) EP 1 498 127 A1

(12) **EUROPEAN PATENT APPLICATION**
published in accordance with Art. 158(3) EPC

(43) Date of publication:
**19.01.2005 Bulletin 2005/03**

(21) Application number: **02807108.2**

(22) Date of filing: **30.09.2002**

(51) Int Cl.[7]: **A61K 31/7056**, A61K 31/4745, A61K 31/282, A61K 31/24, A61K 31/704, A61K 31/7048, A61K 31/519, A61K 45/00, A61P 35/00, A61P 43/00

(86) International application number:
**PCT/JP2002/010186**

(87) International publication number:
**WO 2003/080077 (02.10.2003 Gazette 2003/40)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **26.03.2002 JP 2002084677**

(71) Applicant: **BANYU PHARMACEUTICAL CO., LTD.**
**Chuo-ku, Tokyo 103-8416 (JP)**

(72) Inventors:
- **ARAKAWA,H.**
  **BANYU PHARMA.CO.Ltd.Tsukuba Res.Inst.**
  **Tsukuba-shi,Ibaraki 300-2611 (JP)**
- **MONDEN,Y.**
  **BANYU PHARMACEUTICAL CO., LTD. Business**
  **Chuo-ku, Tokyo 103-8416 (JP)**
- **NAKATSURU,Y.**
  **BANYU PHARMA.CO.LTD.Tsukuba Res.Inst.**
  **Tsukuba-shi, Ibarakai 300-2611 (JP)**
- **KODERA,T.**
  **BANYU PHARMA.CO., LTD.Tsukuba Res.Inst.**
  **Tsukuba-shi, Ibaraki 300-2611 (JP)**

(74) Representative: **Woods, Geoffrey Corlett et al**
**J.A. KEMP & CO.**
**Gray's Inn**
**14 South Square**
**London WC1R 5JJ (GB)**

(54) **USE OF ANTITUMOR INDOLOPYRROLOCARBAZOLE DERIVATIVE AND OTHER ANTICANCER AGENT IN COMBINATION**

(57) This invention relates to a combined preparation for simultaneous, separate, or sequential administration in the treatment of cancer, comprising two separate preparations:

* a preparation comprising, in combination with a pharmaceutically acceptable carrier or diluent, at least one compound of general formula I:

EP 1 498 127 A1

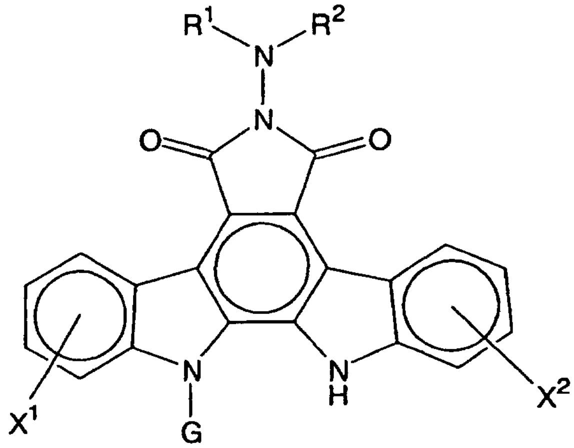

[I]

wherein

$R^1$ and $R^2$ each independently represent a hydrogen atom, lower alkyl, or the like, and G represents pentosyl or the like, $X^1$ and $X^2$ each independently represent a hydrogen atom, a halogen atom, or the like or a pharmaceutically acceptable salt thereof; and

* a preparation, in combination with a pharmaceutically acceptable carrier or diluent, such as antitumor alkylating agents, antitumor antimetabolites, antitumor antibiotics, or plant-derived antitumor agents (a preparation comprising at least one compound of general formula I or a pharmaceutically acceptable salt thereof may be combined with two or more other antitumor agents), and a method for cancer treatment comprising the administration of these preparations in combination.

## Description

Technical Field

The present invention relates to a combined use of an indolopyrrolocarbazole derivative with other antitumor agent(s) which exhibits an excellent effect, particularly a synergistic effect of inhibiting the growth of cancer cells. More particularly, the present invention relates to a combined preparation of an indolopyrrolocarbazole derivative and other antitumor agent(s), a treating method comprising the administration of an indolopyrrolocarbazole derivative and other antitumor agent(s), and use of an indolopyrrolocarbazole derivative and other antitumor agent(s) for manufacturing a preparation for cancer treatment.

Background Art

The development of various pharmaceuticals, particularly, therapeutic agents for infectious diseases, therapeutic agents for hypertension, therapeutic agents for hyperlipemia, and the like has prolonged the human life span. The prolonged life span, however, has increased the death rate from diseases which are considered to be highly associated with aging, and this has become serious issues in developed countries as well as in developing countries. In particular, the death rate from a malignant neoplasm, what is called a cancer, has dramatically increased in Japan, Europe and the United States. There are various methods for cancer treatment, for example, surgery, radiation therapy, chemotherapy, and immune therapy, and the combination of several treating methods has improved the survival ratio. In the treatment of metastasis to other tissues, which is one problem of cancer, however, the treatment by surgery or radiation therapy is limited. Thus, chemotherapy, i.e., medicinal treatment is mainly attempted.

Many agents for chemotherapy have been reported. These antitumor agents are roughly classified into: antitumor alkylating agents that are typified by cyclophosphamide; antitumor antimetabolites that are typified by 5-fluorouracil; antitumor antibiotics that are typified by doxorubicin hydrochloride; plant-derived antitumor agents that are typified by vincristine and paclitaxel; antitumor platinum-complex compounds that are typified by cisplatin; and the like. The present inventors have found a novel indolopyrrolocarbazole derivative having potent antitumor activities, and had applied for patent on the series of compounds (for example, United States Patent Nos. 5591842, 5668271, 5804564, and 5922860, WO 95/30682, WO 96/04293, WO 98/0743, EP-A-0528030, and JP-A-10-245390).

Heretofore, a combined use of staurosporine, i.e., an indolocarbazole derivative, or a derivative thereof with other antitumor agent(s) has been described in Literature (for example, JP-A-02-108636, WO 94/20106, WO 2001/04125, JP-A-08-50012, and JP-A-10-500394). However, staurosporine and its derivatives are significantly different from the compound of general formula I according to the invention of the present application in their structures. Also, while staurosporine and its derivatives exhibit the inhibitory action on protein kinase C, the compound of general formula I according to the present invention exhibits the inhibitory action on topoisomerase I (T. Yoshinari et al., Cancer Research, 55, 1310-1315 (1995)). Further, the prior art documents do not disclose or suggest the combined use of the compound of general formula I according to the present invention with other antitumor agent(s).

Disclosure of the Invention

The present invention is aimed to expand the application range of chemotherapeutics by combining an antitumor indolocarbazole derivative with other antitumor agent(s) and to provide a better antitumor effect, in particular, a synergistic effect compared to the use of one agent.

More specifically, an object of the present invention is to provide a combined preparation or mixture for synergistically enhancing the antitumor effects, which comprises an indolopyrrolocarbazole derivative in combination with other antitumor agent(s). Another object of the present invention is to treat cancer patients by administering the indolopyrrolocarbazole derivative in combination with other antitumor agent(s). A further object of the present invention is to use the indolopyrrolocarbazole derivative and other antitumor agent(s) to produce a preparation for cancer treatment.

The present inventors had conducted concentrated studies on combinations of the already disclosed indolopyrrolocarbazole antitumor compound with other antitumor agent(s). As a result, they found a combination exhibiting an excellent antitumor effect, in particular, a synergistic effect.

More specifically, the present invention relates to a combined preparation for simultaneous, separate, or sequential administration in the treatment of cancer, comprising two separate preparations:

* a preparation comprising, in combination with a pharmaceutically acceptable carrier or diluent, at least one compound of general formula I:

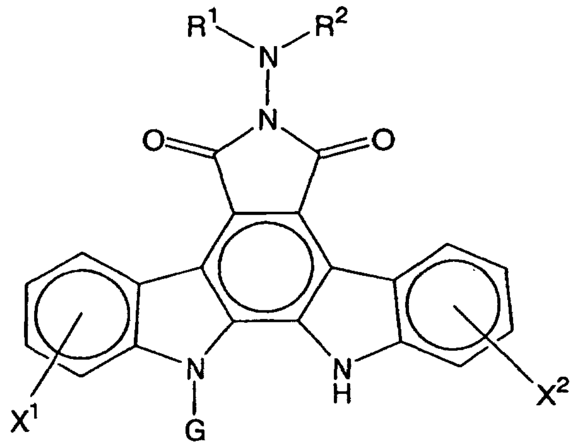

[I]

[wherein $R^1$ and $R^2$ each independently represent:

a hydrogen atom, lower alkyl, lower alkenyl, lower alkynyl, aryl, aralkyl, or heterocyclic group (wherein the lower alkyl, the lower alkenyl, the lower alkynyl, the aryl, the aralkyl, and the heterocyclic group may each have one to five of the same or different substituents selected from the group consisting of carboxyl, carbamoyl, sulfo, amino, cyano, mono-lower alkylamino, di-lower alkylamino, hydroxyl, and a halogen atom);
a group of formula $-Y-R^3$ {wherein Y represents carbonyl, thiocarbonyl, or sulfonyl, and $R^3$ represents a hydrogen atom, lower alkyl, cycloalkyl, cycloalkyl-lower alkyl, aryl, aralkyl, lower alkoxy, hydrazino, amino, arylamino, carbamoyl, or heterocyclic group (wherein the lower alkyl, the cycloalkyl, the cycloalkyl-lower alkyl, the aryl, the aralkyl, and the heterocyclic group may each have one to four of the same or different substituents selected from the group consisting of a halogen atom, optionally protected hydroxyl, amino, carboxyl, carbamoyl, cyano, and lower alkoxycarbonyl in which the amino and the carbamoyl may each be further mono- or di-substituted by lower alkyl optionally substituted by a substituent or substituents selected from the group consisting of a halogen atom, hydroxyl, amino, carboxyl, carbamoyl, and lower alkoxycarbonyl)}; or
a group of formula $-(CH_2)_m-R^4$ (wherein $R^4$ is pyridyl, furyl, or thienyl (wherein the pyridyl, the furyl, and the thienyl may each have one or two substituents selected from the group consisting of hydroxyl, lower alkoxy, hydroxy-lower alkyl, and hydroxy-lower alkenyl), and m is an integer of 1 to 3},
$R^1$ and $R^2$ are combined together to represent lower alkylidene (wherein the lower alkylidene may have one to four of the same or different substituents selected from the group consisting of amino, mono-lower alkylamino, di-lower alkylamino, hydroxyl, carboxyl, and sulfo), or
$R^1$ and $R^2$, together with the nitrogen atom to which they bind, form heterocyclic group (wherein the heterocyclic group may have, on the ring, lower alkyl optionally substituted by a group or groups selected from the group consisting of amino, hydroxyl, carboxyl, and sulfo),
G represents a pentosyl or hexosyl, and
$X^1$ and $X^2$ each independently represent a hydrogen atom, a halogen atom, amino, mono-lower alkylamino, di-lower alkylamino, hydroxyl, lower alkoxy, aralkoxy, carboxyl, lower alkoxycarbonyl, or lower alkyl] or a pharmaceutically acceptable salt thereof; and

* a preparation comprising, in combination with a pharmaceutically acceptable carrier or diluent, at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents or a pharmaceutically acceptable salt thereof

(wherein the antitumor alkylating agents are nitrogen mustard N-oxide, cyclophosphamide, ifosfamide, melphalan, busulfan, mitobronitol, carboquone, thiotepa, ranimustine, nimustine, or temozolomide,

the antitumor antimetabolites are methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, or fludarabine,

the antitumor antibiotics are actinomycin D, doxorubicin, daunorubicin, neocarzinostatin, bleomycin, peplomycin, mitomycin C, aclarubicin, pirarubicin, epirubicin, zinostatin stimalamer, or idarubicin,

the plant-derived antitumor agents are vincristine, vinblastine, vindeshine, etoposide, sobuzoxane, docetaxel, paclitaxel, or vinorelbine,
the antitumor platinum-complex compounds are cisplatin, carboplatin, nedaplatin, or oxaliplatin,
the antitumor campthotecin derivatives are irinotecan, topotecan, or campthotecin,
the antitumor tyrosine kinase inhibitors are Iressa or SU5416,
the monoclonal antibodies are IMC-C225, RhuMabVEGF, or Rituximab,
the interferons are interferon $\alpha$, interferon $\alpha$-2a, interferon $\alpha$-2b, interferon $\beta$, interferon $\gamma$-1a, or interferon $\gamma$-n1,
the biological response modifiers are krestin, lentinan, sizofiran, picibanil, or ubenimex, and
the other antitumor agents are mitoxantrone, L-asparaginase, procarbazine, dacarbazine, hydroxycarbamide, pentostatin, or tretinoin). Doxorubicin may be hereinafter referred to as "adriamycin."

More specifically, the present invention relates to a combined preparation for simultaneous, separate, or sequential administration in the treatment of cancer, comprising two separate preparations:

* a preparation comprising, in combination with a pharmaceutically acceptable carrier or diluent, at least one compound of general formula I as defined above (wherein $R^1$, $R^2$, $R^3$, $R^4$, m, Y, G, $X^1$, and $X^2$ are the same as defined in Claim 1) or a pharmaceutically acceptable salt thereof; and
* a preparation comprising, in combination with a pharmaceutically acceptable carrier or diluent, at least one antitumor agent selected from the group consisting of: 5-fluorouracil; S-1; gemcitabine; doxorubicin and etoposide; docetaxel and paclitaxel; cisplatin, carboplatin, and oxaliplatin; irinotecan, topotecan, and campthotecin; Iressa and SU5416; and IMC-C225 and RhuMabVEGF or a pharmaceutically acceptable salt thereof (wherein, if the preparation contains 5-fluorouracil, the preparation may further contain leucovorin or may be combined with a separate leucovorin preparation).

Particularly preferably, the present invention relates to a combined preparation for simultaneous, separate, or sequential administration in the treatment of cancer, comprising two separate preparations:

* a preparation comprising, in combination with a pharmaceutically acceptable carrier or diluent, a compound of formula IA:

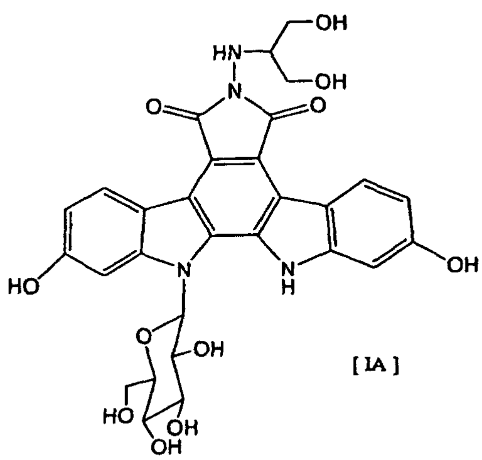

[IA]

(this compound is hereinafter referred to as "compound A") or a pharmaceutically acceptable salt thereof; and
* a preparation comprising, in combination with a pharmaceutically acceptable carrier or diluent, an antitumor agent selected from the group consisting of 5-fluorouracil, doxorubicin, cisplatin, carboplatin, irinotecan, topotecan, and campthotecin (wherein, if this preparation contains 5-fluorouracil, it may be combined with a separate leucovorin preparation) or a pharmaceutically acceptable salt thereof. "Compound A" is 6-N- (1-hydroxymethyl-2-hydroxy) ethylamino-12,13-dihydro-2,10-dihydroxy-12-($\beta$-D-glucopyranosyl)-5H-indolo[2,3-a]-pyrrolo[3,4-c]-carbazole-5,7 (6H)-dione.

The present invention also relates to a method for cancer treatment comprising the administration of a therapeutically effective amount of at least one compound of general formula I (wherein $R^1$, $R^2$, $R^3$, $R^4$, m, Y, G, $X^1$, and $X^2$ are the same as defined in Claim 1) or a pharmaceutically acceptable salt thereof in combination with a therapeutically effective amount of at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents (wherein a definition of each antitumor agent is the same as above) or a pharmaceutically acceptable salt thereof.

More particularly, the present invention relates to a method for cancer treatment comprising the administration of a therapeutically effective amount of at least one compound of general formula I (wherein $R^1$, $R^2$, $R^3$, $R^4$, m, Y, G, $X^1$, and $X^2$ are the same as above) or a pharmaceutically acceptable salt thereof in combination with a therapeutically effective amount of at least one antitumor agent selected from the group consisting of: 5-fluorouracil; S-1; gemcitabine; doxorubicin and etoposide; docetaxel and paclitaxel; cisplatin, carboplatin, and oxaliplatin; irinotecan, topotecan, and campthotecin; Iressa and SU5416; and IMC-C225 and RhuMabVEGF or a pharmaceutically acceptable salt thereof (wherein, if the compound of general formula I is combined with 5-fluorouracil, leucovorin may be further combined).

Particularly preferably, the present invention relates to a method for cancer treatment comprising the administration of a therapeutically effective amount of compound A or a pharmaceutically acceptable salt thereof in combination with a therapeutically effective amount of an antitumor agent selected from the group consisting of 5-fluorouracil, doxorubicin, cisplatin, carboplatin, irinotecan, topotecan, and campthotecin or a pharmaceutically acceptable salt thereof (wherein, if 5-fluorouracil is combined with compound A, leucovorin may be further combined).

Further, the present invention relates to the use of at least one compound of general formula I (wherein $R^1$, $R^2$, $R^3$, $R^4$, m, Y, G, $X^1$, and $X^2$ are the same as above) or a pharmaceutically acceptable salt thereof and at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents (wherein a definition of each antitumor agent is the same as above) or a pharmaceutically acceptable salt thereof, for manufacturing a preparation for cancer treatment.

More particularly, the present invention relates to the use of at least one compound of general formula I (wherein $R^1$, $R^2$, $R^3$, $R^4$, m, Y, G, $X^1$, and $X^2$ are the same as defined in Claim 1) or a pharmaceutically acceptable salt thereof and at least one antitumor agent selected from the group consisting of 5-fluorouracil; S-1; gemcitabine; doxorubicin and etoposide; docetaxel and paclitaxel; cisplatin, carboplatin, and oxaliplatin; irinotecan, topotecan, and campthotecin; Iressa and SU5416; IMC-C225 and RhuMabVEGF or a pharmaceutically acceptable salt thereof (wherein, if the compound of general formula I and 5-fluorouracil are used, leucovorin may be further used), for manufacturing a preparation for cancer treatment.

Particularly preferably, the present invention relates to the use of compound A or a pharmaceutically acceptable salt thereof and an antitumor agent selected from the group consisting of 5-fluorouracil, doxorubicin, cisplatin, carboplatin, irinotecan, topotecan, and campthotecin or a pharmaceutically acceptable salt thereof (wherein, if the compound of general formula I and 5-fluorouracil are used, leucovorin may be further used), for manufacturing a preparation for cancer treatment.

Further, the present invention relates to a pharmaceutical composition comprising, in combination with a pharmaceutically acceptable carrier or diluent, at least one compound of general formula I (wherein $R^1$, $R^2$, $R^3$, $R^4$, m, Y, G, $X^1$, and $X^2$ are the same as defined above) or a pharmaceutically acceptable salt thereof and at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, biological response modifiers, and other antitumor agents (wherein a definition of each antitumor agent is the same as defined above) or a pharmaceutically acceptable salt thereof.

More particularly, the present invention relates to a pharmaceutical composition comprising, in combination with a pharmaceutically acceptable carrier or diluent, at least one compound of general formula I (wherein $R^1$, $R^2$, $R^3$, $R^4$, m, Y, G, $X^1$, and $X^2$ are the same as defined above) or a pharmaceutically acceptable salt thereof and at least one antitumor agent selected from the group consisting of 5-fluorouracil; S-1; gemcitabine hydrochloride; doxorubicin hydrochloride and etoposide; docetaxel hydrate and paclitaxel; cisplatin, carboplatin, and oxaloplatin; irinotecan, topotecan, and campthotecin; Iressa and SU5416; IMC-C225 and RhuMabVEGF or a pharmaceutically acceptable salt thereof (wherein, if the composition contains the compound of general formula I and 5-fluorouracil, it may further contain leucovorin).

Particularly preferably, the present invention relates to a pharmaceutical composition comprising, in combination with a pharmaceutically acceptable carrier or diluent, compound A or a pharmaceutically acceptable salt thereof and an antitumor agent selected from the group consisting of 5-fluorouracil, doxorubicin, cisplatin, carboplatin, irinotecan, topotecan, and campthotecin or a pharmaceutically acceptable salt thereof (wherein, if the compound of general

formula I and 5-fluorouracil are contained, leucovorin may be further contained).

The term "lower" used herein refers to the group or compound, to which this term "lower" has been applied, having 6 or fewer, preferably 4 or fewer carbon atoms.

"Lower alkyl" refers to linear or branched alkyl having one to six carbon atoms, and examples thereof include methyl, ethyl, propyl, isopropyl, butyl, isobutyl, sec-butyl, tert-butyl, pentyl, isopentyl, neopentyl, and hexyl.

"Lower alkenyl" refers to linear or branched alkenyl having three to six carbon atoms, and examples thereof include propenyl, 2-butenyl, 3-butenyl, 3-pentenyl, and 4-hexenyl.

"Lower alkynyl" refers to linear or branched alkynyl having three to six carbon atoms, and examples thereof include propynyl, 2-butynyl, 3-butynyl, 3-pentynyl, and 4-hexynyl.

"Cycloalkyl" refers to cycloalkyl having three to six carbon atoms, and examples thereof include cyclopropyl, cyclobutyl, cyclopentyl, and cyclohexyl.

"Cycloalkyl-lower alkyl" refers to "lower alkyl" substituted by "cycloalkyl" in which the cycloalkyl and lower alkyl moieties are as defined above, and examples thereof include cyclopropylmethyl, cyclobutylmethyl, cyclopentylmethyl, cyclohexylmethyl, 1-cyclopropylethyl, 2-cyclopropylethyl, 1-cyclobutylethyl, 2-cyclobutylethyl, 1-cyclopentylethyl, 2-cyclopentylethyl, 1-cyclohexylethyl, 3-cyclohexylpropyl, 3-cyclopentylpropyl, 4-cyclohexylbutyl, and 4-cyclopentylbutyl. The cycloalkyl-lower alkyl preferably has 4 to 10 carbon atoms in total.

"Aryl" may be monocycle or polycycle, and examples thereof include aryl having six to twelve carbon atoms such as phenyl, naphthyl, and tetrahydronaphthyl.

"Aralkyl" refers to "lower alkyl" substituted by "aryl" in which the aryl and lower alkyl moieties are as defined above, and examples thereof include aralkyl having seven to fifteen carbon atoms such as benzyl, phenethyl, phenylpropyl, phenylbutyl, phenylpentyl, naphthylmethyl, and naphthylethyl.

"Heterocyclic group" includes 5- or 6-membered heterocyclic group containing 1 to 4 hetero atoms selected from the group consisting of a nitrogen atom, an oxygen atom, and a sulfur atom. Examples thereof include: aromatic heterocyclic group such as pyrrolyl, furyl, thienyl, oxazolyl, isoxazolyl, thiazolyl, isothiazolyl, imidazolyl, pyrazolyl, oxadiazolyl, thiadiazolyl, triazolyl, tetrazolyl, furazanyl, pyridyl, pyridazinyl, pyrimidinyl, pyrazinyl, and triazinyl; and nonaromatic heterocyclic groups such as dihydrothienyl, tetrahydrothienyl, pyrrolinyl, pyrrolidinyl, imidazolidinyl, imidazolinyl, piperidinyl, piperadinyl, oxazolinyl, oxazolydinyl, isoxazolinyl, isoxazolidinyl, thiazolinyl, thiazolidinyl, isothiazolinyl, isothiazolidinyl, 1,2-dithiolanyl, 1,3-dithiolanyl, 1,2-dithiolyl, 1,3-dithiolyl, dihydrothiopyranyl, tetrahydrothiopyranyl, 1,4-dithianyl, 1,4-dithiinyl, 1,4-oxathiinyl, and thiomorpholinyl.

Examples of "mono-lower alkylamino" include methylamino, ethylamino, propylamino, isopropylamino, butylamino, pentylamino, and hexylamino. Examples of "di-lower alkylamino" include dimethylamino, ethylmethylamino, diethylamino, ethylpropylamino, dipropylamino, butylmethylamino, dibutylamino, butylethylamino, methylpentylamino, hexylmethylamino, and ethylhexylamino.

"Arylamino" refers to amino that is substituted by aryl as defined above, and examples thereof are phenylamino and naphthylamino.

A "halogen atom" includes fluorine, chlorine, bromine, and iodine atoms.

Examples of "lower alkylidene" include linear or branched alkylidene having one to six carbon atoms such as methylene, ethylidene, propylidene, isopropylidene, butylidene, isobutylidene, sec-butylidene, pentylidene, isopentylidene, neopentylidene, and hexylidene.

"Lower alkoxy" refers to "lower alkyl" -O- group in which the lower alkyl moiety is as defined above, and refers to linear or branched alkoxy having one to six carbon atoms, preferably one to four carbon atoms, more preferably one or two carbon atoms, and most preferably one carbon atom. Examples thereof include methoxy, ethoxy, propoxy, isopropoxy, n-butoxy, isobutoxy, sec-butoxy, tert-butoxy, pentoxy, isopentoxy, neopentoxy, and hexoxy.

"Lower alkoxycarbonyl" refers to "lower alkoxy" -CO- group in which the lower alkoxy moiety is as defined above. Examples thereof include methoxycarbonyl, ethoxycarbonyl, propyloxycarbonyl, isopropyloxycarbonyl, butyloxycarbonyl, isobutyloxycarbonyl, pentyloxycarbonyl, and hexyloxycarbonyl.

"Aralkoxy" refers to "lower alkoxy" substituted by "aryl" in which the aryl and lower alkoxy moieties are as defined above. Examples thereof include benzyloxy, phenethyloxy, phenylpropoxy, α-naphthylmethoxy, β-naphthylmethoxy, naphthylethoxy, and tetrahydronaphthylmethoxy.

Examples of a protective group in "optionally protected hydroxyl" include: $C_{2-6}$ alkanoyl such as acetyl, propionyl, and butyryl; aroyl such as benzoyl; substituted or unsubstituted aralkyl such as benzyl and 4-methoxybenzyl; and an acetal-forming group such as acetonide.

"Hydroxy-lower alkyl" refers to linear or branched hydroxyalkyl one to six carbon atoms, preferably one to four carbon atoms, and more preferably two or three carbon atoms, and examples thereof include hydroxymethyl, hydroxyethyl, hydroxypropyl, hydroxybutyl, and hydroxyhexyl.

"Hydroxy-lower alkenyl" refers to linear or branched hydroxyalkenyl two to six carbon atoms, preferably three or four carbon atoms, and more preferably three carbon atoms, and examples thereof include 3-hydroxy-1-propenyl.

m is an integer of 1 to 3, and preferably 1.

"Pentosyl" and "hexosyl" refer to pentosyl and hexosyl in which hydroxyl is optionally replaced by one to three of the same or different groups selected from the group consisting of a hydrogen atom, lower alkyl, lower alkylcarbonyloxy, lower alkoxy, and amino, or is optionally oxidized. Examples thereof include: groups derived from pentose such as ribose, arabinose, xylose, and 2-deoxyribose and groups derived from hexose such as allose, glucose, mannose, galactose, glucosamine, galactosamine, 2-deoxyglucose, 4-O-methylglucose, rhamnose, and glucuronic acid. Preferable examples thereof include β-D-glucopyranosyl.

In the compound of general formula I, G preferably represents the group of formula:

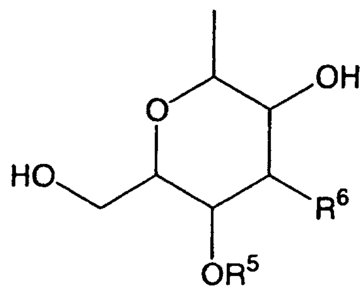

or

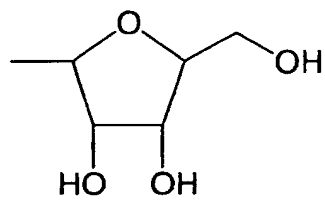

wherein $R^5$ represents a hydrogen atom or lower alkyl, and $R^6$ represents hydroxyl or amino.

In the compound of general formula I, $X^1$ and $X^2$ bind preferably to the indolopyrrolocarbazole ring at the 1- or 2-position and at the 10- or 11-position, respectively, and each independently represent a halogen atom, hydroxyl, lower alkoxy, or aralkoxy.

In the compound of general formula I, preferably, G is β-D-glucopyranosyl, and $X^1$ and $X^2$ represent hydroxyl bonded to the indolopyrrolocarbazole ring at the 2-position and at the 10-position, respectively.

In the compound of general formula I, preferably, $R^1$ represents a hydrogen atom, and $R^2$ represents a group of the formula:

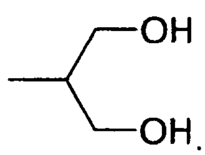

In the compound of general formula I, preferably, $R^1$ represents a hydrogen atom, and $R^2$ represents $-CH_2-R^4$ wherein $R^4$ represents 6-hydroxymethylpyridin-2-yl.

In the compound of general formula I, preferably, $R^1$ represents a hydrogen atom, and $R^2$ represents $-CH_2-R^4$ wherein $R^4$ represents pyridin-4-yl.

In the compound of general formula I, preferably, $R^1$ represents a hydrogen atom, and $R^2$ represents $-CH_2-R^4$ wherein $R^4$ represents 5-hydroxymethylpyridin-4-yl.

Preferably, preferred embodiments of G, $X^1$ and $X^2$, and $R^1$ and $R^2$ are optionally combined.

The compound of general formula I according to Claim 1 is preferably a compound of formula IA (i.e., "com-

pound A"):

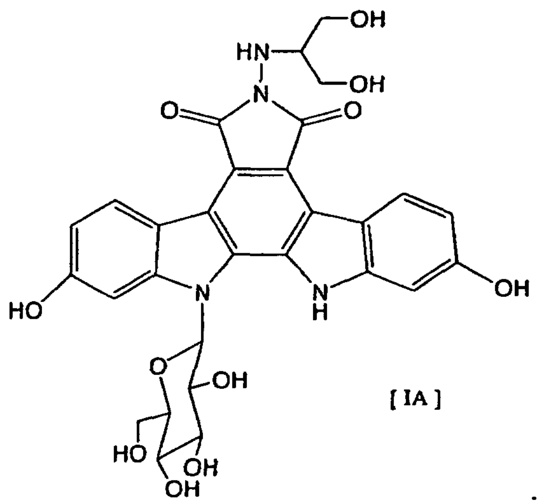

[IA]

.

The compound of general formula I according to the present invention can be present in the form of a pharmaceutically acceptable salt. Examples of such a salt include acid addition salts with inorganic acids such as hydrochloric acid and sulfuric acid or acid addition salts with organic acids such as acetic acid, citric acid, tartaric acid, and maleic acid. When the compound of the present invention contains an acidic group, the acidic group can be present in the form of: alkali metal salts such as potassium salt and sodium salt; alkaline earth metal salts such as magnesium salt and calcium salt; and salts with organic bases such as ethylamine salt and arginine salt.

Those skilled in the art can easily produce the compound of general formula I in accordance with the methods described in known literatures such as United State Patent Nos. 5591842, 5668271, 5804564, and 5922860 or the similar methods thereto.

The term "pharmaceutically acceptable carrier or diluent" used herein includes: solvents such as water, physiological saline, alcohol (e.g., ethanol), glycerin, and vegetable oil; and additives such as excipient, base, disintegrator, binder, lubricant, wetting agent, stabilizer, emulsifier, dispersant, preservative, sweetening agent, coloring agent, corrigent, aromatic, buffer, solubilizer, antiseptic agent, salt for changing the osmotic pressure, coating agent, and antioxidant.

The term "treatment of cancer" used herein refers to inhibition of cancer cell growth by administering an antitumor agent to a cancer patient. Preferably, this treatment enables the regression of cancer growth. Specifically, the size of measurable cancer can be decreased. More preferably, this treatment can completely eliminate cancer.

"Cancer" used herein refers to solid cancer and hematopoietic cancer. Examples of solid cancer include cerebral tumor, head and neck cancer, esophageal cancer, thyroid cancer, small cell lung cancer, non-small cell lung cancer, breast cancer, stomach cancer, gallbladder and bile duct cancer, liver cancer, pancreas cancer, colon cancer, rectal cancer, ovarian cancer, choriocarcinoma, uterine cancer, cervical cancer, renal pelvic and ureteral cancer, bladder cancer, prostate cancer, penile cancer, testicular cancer, embryonal cancer, Wilms' tumor, skin cancer, malignant melanoma, neuroblastoma, osteosarcoma, Ewing's tumor, and soft tissue sarcoma,; preferably, colon cancer, small cell lung cancer, non-small cell lung cancer, bladder cancer, head and neck cancer, stomach cancer, pancreas cancer, liver cancer, and ovarian cancer; more preferably colon cancer, non-small cell lung cancer, and head and neck cancer. Examples of hematopoietic cancer include acute leukemia, chronic lymphatic leukemia, chronic myelocytic leukemia, polycythemia vera, malignant lymphoma, multiple myeloma, and non Hodgkin's lymphoma.

The term "preparation" used herein includes oral preparations and parenteral preparations. Examples of oral preparations include tablets, capsules, powders, and granules. Examples of parenteral preparations include sterilized liquid preparations such as a solution or suspension; and specifically the examples are injections and infusions; preferably, intravenous injections or infusions; more preferably intravenous infusions.

The term "combined preparation" used herein refers to those comprising two or more preparations for simultaneous, separate, or sequential administration in the treatment. They may be a so-called kit type preparation or pharmaceutical composition. The "combined preparation" include those prepared by further combining at least one preparation with the combined preparation comprising two separate preparations used in the treatment of cancer.

The two separate preparations mentioned above can be further combined with, in combination with a pharmaceutically acceptable carrier or diluent, at least one preparation comprising at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents (wherein a definition of each antitumor agent is the same as defined in Claim 1) or a pharmaceutically acceptable salt thereof. In this case, the above-mentioned at least one preparation further added and the two separate preparation above may be administered simultaneously, separately, or sequentially. For example, a combined preparation comprising three preparations includes a preparation comprising the compound of general formula I, a preparation comprising 5-fluorouracil, and a preparation comprising leucovorin.

One of or both of the two separate preparations in the combined preparation may be a parenteral preparation, which may be preferably injections or infusions, and more preferably intravenous infusions.

In general, the "preparation" according to the present invention may comprise a therapeutically effective amount of the compound of the present invention in combination with a pharmaceutically acceptable carrier or diluent. This technique of formulation is considered to be general technical knowledge and well known to those skilled in the art. Preferably, the preparations for intravenous infusions or injections can be prepared in combination with a pharmaceutically acceptable carrier or diluent by various methods that are well known to those skilled in the art.

Further, when the combined preparation of the present invention is used, the term "administration" used herein refers to parenteral and/or oral administration, and is preferably parenteral administration. Specifically, when the combined preparation is administered: both administrations may be parenteral; one administration may be parenteral while the other may be oral; or both administrations may be oral. Preferably, both preparations relating to the combined preparation is administered parenterally. "Parenteral administration" is, for example, intravenous administration, subcutaneous administration, or intramuscular administration, with intravenous administration being preferred. When three or more preparations are combined and administered, at least one preparation may be parenterally administered. Preferably, it may be intravenously administered; and more preferably, it may be subject to intravenous infusion or injection.

In the implementation of the present invention, the compound of general formula I may be administered simultaneously with other antitumor agents. The compound of general formula I may be first administered followed by the administration of other antitumor agent(s). Alternatively, other antitumor agent(s) may be first administered followed by the administration of the compound of general formula I. Further, the compound of general formula I may be administered, and then other antitumor agent(s) may be separately administered after a while. Alternatively, other antitumor agent(s) may be administered, and then the compound of general formula I may be separately administered after a while. The order and the time interval for the administration may be suitably selected by those skilled in the art in accordance with, for example, a preparation comprising the compound of general formula I to be used, a preparation comprising an antitumor agent that is used therewith, a type of cancer cells to be treated, and a condition of the patient.

The term "simultaneously" used herein refers to the use of preparations for the treatment at substantially the same time. The term "separately" refers to the separate use of preparations for the treatment at different times; for example, one agent is used on the first day and another agent is used on the second day. The term "sequentially" refers to the use of preparations in order; for example, one agent is first used and another agent is used for the treatment after a determined length of time.

The "antitumor alkylating agents" used herein refer to alkylating agents having antitumor activities in which the "alkylating agents" generally refer to those giving alkyl in the alkylation reaction for substituting a hydrogen atom of an organic compound with alkyl. The "antitumor alkylating agents" include, for example, nitrogen mustard N-oxide, cyclophosphamide, ifosfamide, melphalan, busulphan, mitobronitol, carboquone, thiotepa, ranimustine, nimustine, and temozolomide (3-methyl-4-oxo-3,4-dihydroimidazo[5,1-d][1,2,3,5]tetrazine-8-carboxamide), with cyclophosphamide being preferred.

The "antitumor antimetabolites" used herein refer to antimetabolites having antitumor activities in which "antimetabolites" generally include the substances which disturb normal metabolism, and the substances which inhibit the electron transfer system to prevent energy-rich intermediates from being produced, due to their structural or functional similarities to metabolites that are important for living organisms (e.g., vitamin, coenzyme, amino acid, and sugars). Examples of "antitumor antimetabolites" include methotrexate, 6-mercaptopurine riboside, mercaptopurine, 5-fluorouracil (5-FU), tegafur, UFT, doxifluridine, carmofur (hexylcarbamoyl-5-FU), cytarabine (cytosine arabinoside), cytarabine ocfosfate, enocitabine (behenoyl-ara-C), S-1, gemcitabine, and fludarabine, with 5-FU, S-1, and gemcitabine being preferred. 5-FU is preferably used with leucovorin.

The "antitumor antibiotics" used herein refer to antibiotics having antitumor activities in which the "antibiotics" include substances that are produced by microorganisms and inhibit cell growth and other functions of microorganisms and other living organisms. Examples of "antitumor antibiotics" include actinomycin D (dactinomycin), doxorubicin (adriamycin), daunorubicin (daunomycin), neocarzinostatin, bleomycin, peplomycin, mitomycin C, aclarubicin (aclacinomycin A), pirarubicin, epirubicin, zinostatin stimalamer, and idarubicin, with doxorubicin being preferred.

The "plant-derived antitumor agents" used herein include a compound having antitumor activities which originated from plants or a compound prepared by giving chemical modification to the compound. The "plant-derived antitumor agents" include, for example, vincristine, vinblastine, vindeshine, etoposide, sobuzoxane, docetaxel, paclitaxel, and vinorelbine, with etoposide, docetaxel, and paclitaxel being preferred.

The "antitumor campthotecin derivatives" used herein include campthotecin itself, and refer to a compound that is structurally associated with campthotecin, directed for inhibiting cancer cell growth. Examples of the "antitumor campthotecin derivatives" include, but are not particularly limited to, campthotecin, 10-hydroxycampthotecin, topotecan, irinotecan, and 9-aminocampthotecin, with campthotecin, topotecan, and irinotecan being preferred. Irinotecan is metabolized *in vivo* to exhibit antitumor effects as SN-38. The action mechanism and the activity of the campthotecin derivatives are considered to be substantially the same as those of campthotecin (e.g., Nitta et al., Gan to Kagaku Ryouhou (Cancer and Chemotherapy), 14, 850-857 (1987)).

The "antitumor platinum-complex compounds" used herein refer to platinum-complex compounds having antitumor activities in which "platinum-complex compounds" refer to platinum-complex compounds which give platinum in the form of ion. Preferable platinum compounds include: cisplatin; cis-diamminediaquoplatinum (II)-ion; chloro(diethylenetriamine)-platinum (II) chloride; dichloro(ethylenediamine)-platinum (II); diammine(1,1-cyclobutanedicarboxylato)platinum (II) (carboplatin); spiroplatin; iproplatin; diammine(2-ethylmalonato)-platinum (II); ethylenediaminemalonatoplatinum (II); aqua(1,2-diaminodicyclohexane)sulfatoplatinum (II); aqua(1,2-diaminodicyclohexane)malonatoplatinum (II); (1,2-diaminocyclohexane)malonatoplatinum (II); (4-carboxyphthalato)(1,2-diaminocyclohexane) platinum (II); (1,2-diaminocyclohexane)-(isocitrato)platinum (II); (1,2-diaminocyclohexane)oxalato platinum (II); ormaplatin; tetraplatin; carboplatin; nedaplatin; and oxaliplatin. Preferable platinum compounds include cisplatin (i.e., cys-dichlorodiammineplatinum (II)), carboplatin, and oxaliplatin. Cisplatin is commercially available. Other antitumor platinum-complex compounds exemplified herein are known, commercially available, and/or producible by those skilled in the art by conventional techniques.

The "antitumor tyrosine kinase inhibitors" refer to tyrosine kinase inhibitors having antitumor activities in which the " tyrosine kinase inhibitors" refer to chemical substances that inhibit "tyrosine kinase," which transfers γ-phosphate in ATP to hydroxyl of the specific tyrosine in a protein. Examples of "antitumor tyrosine kinase inhibitors" include: Iressa (ZD-1839; N-(3-chloro-4-fluorophenyl)-7-methoxy-6-[3-(4-morpholinyl)propoxy]-4-quinazolinamine) and SU5416 (Semaxanib; (3Z)-3-[(3,5-dimethyl-1H-pyrrol-2-yl)methylene]-1,3-dihydro-2H-indol-2-one).

The "monoclonal antibodies" are also referred to as "monoclonal antibodies," and refer to antibodies produced by antibody-producing cells of a monoclone, and examples thereof include IMC-C225 (Cetuximab), RhuMabVEGF (Bevacizumab), and Rituximab.

The "interferons" refer to interferons having antitumor activities in which the "interferons" generally refer to glycoproteins having a molecular weight of about 20,000 that are produced and secreted by almost all of animal cells upon virus infection, and, in addition to inhibition of virus growth, have various immune effector mechanisms such as inhibition of cell (particularly tumor cell) growth and enhancement of natural killer activities, and are designated as one type of cytokine. Examples of "interferons" include interferon α, interferon α-2a, interferon α-2b, interferon β, interferon γ-1a, and interferon γ-n1.

In general, the "biological response modifiers" are also referred to as "biological response modifiers (BRM)" that are generic terms for substances or agents for modifying defense mechanisms of living organisms or biological responses such as survival, growth, or differentiation of tissue cells to direct them to be useful for individuals against tumor, infection, or other diseases. Examples of "biological response modifiers" include krestin, lentinan, sizofiran, picibanil, and ubenimex.

The "other antitumor agents" refer to those which do not belong to any of the above antitumor agents having antitumor activities. Examples of "other antitumor agents" include mitoxantrone, L-asparaginase, procarbazine, dacarbazine, hydroxycarbamide, pentostatin, and tretinoin.

The "antitumor alkylating agents," "antitumor antimetabolites," "antitumor antibiotics," "plant-derived antitumor agents," "antitumor platinum-complex compounds," "antitumor campthotecin derivatives," "antitumor tyrosine kinase inhibitors," "monoclonal antibodies," "interferons," "biological response modifiers," and "other antitumor agents" are publicly known" commercially available, or can be prepared by those skilled in the art by well-known or conventional methods. The manufacturing process of SU-5416 is described in, for example, United States Patent No. 5,792,783; that of Iressa is described in, for example, United States Patent No. 5,770,599; that of IMC-C225 is described in, for example, WO 96/40210; that of RhuMab-VEGF is described in, for example, WO 94/10202; that of oxaliplatin is described in, for example, United States Patent Nos. 5,420,319 and 5,959,133; that of gemcitabine is described in, for example, United States Patent Nos. 5,434,254 and 5,223,608; that of campthotecin is described in United States Patent Nos. 5,162,532, 5,247,089, 5,191,082, 5,200,524, 5,243,050, and 5,321,140; that of irinotecan is described in, for example, United States Patent No. 4,604,463; that of topotecan is described in, for example, United States Patent No. 5,734,056; that of temozolomide is described in, for example, JP-B-4-5029; and that of Rituximab is described in JP-A-2-503143.

The antitumor alkylating agents mentioned above are commercially available: for example, nitrogen mustard N-oxide as Nitromin (trade name, Mitsubishi Pharma Corporation); cyclophosphamide as Endoxan (trade name, Shionogi & Co., Ltd.); ifosfamide as Ifomide (trade name, Shionogi & Co., Ltd.); melphalan as Alkeran (trade name, GlaxoSmithKline); busulphan as Mablin (trade name, Takeda Chemical Ind.); mitobronitol as Myebrol (trade name, Kyorin Pharmaceutical); carboquone as Esquinon (trade name, Sankyo Co); thiotepa as Tespamin (trade name, Sumitomo Pharmaceuticals Co., Ltd.); ranimustine as Cymerin (trade name, Mitsubishi Pharma Corporation); and nimustine as Nidran (trade name, Sankyo Co).

The antitumor antimetabolites mentioned above are commercially available: for example, methotrexate as Methotrexate (trade name, Takeda Chemical Ind.); 6-mercaptopurine riboside as Thioinosie (trade name, Aventis); mercaptopurine as Leukerin (trade name, Takeda Chemical Ind.); 5-fluorouracil as 5-FU (trade name, KYOWA HAKKO KOGYO Co., Ltd.); tegafur as Futraful (trade name, TAIHO Pharmaceutical Co., Ltd.); doxifluridine as Furtulon (trade name, Nippon Roche K.K.); carmofur as Yamafur (trade name, Yamanouchi Pharmaceutical); cytarabine as Cylocide (trade name, Nippon Shinyaku); cytarabine ocfosfate as Starasid (trade name, Nippon Kayaku); enocitabine as Sunrabin (trade name, ASAHI KASEI CORP); S-1 as TS-1 (trade name, TAIHO Pharmaceutical Co., Ltd.); gemcitabine (Qventas, U.S.A.); and fludarabine as Fludara (trade name, Nihon Schering).

The antitumor antibiotics mentioned above are commercially available: for example, actinomycin D as Cosmegen (trade name, BANYU PHARMACEUTICAL CO., LTD.); doxorubicin as Adriacin (trade name, KYOWA HAKKO KOGYO Co., Ltd.); daunorubicin as Daunomycin (trade name, MEIJI SEIKA KAISHA, LTD.); neocarzinostatin as Neocarzinostatin (trade name, Yamanouchi Pharmaceutical); bleomycin as Bleo (trade name, Nippon Kayaku); peplomycin as Pepleo (trade name, Nippon Kayaku); mitomycin C as Mitomycin (trade name, KYOWA HAKKO KOGYO Co., Ltd.); aclarubicin as Aclacinon (trade name, Yamanouchi Pharmaceutical); pirarubicin as Pinorubin (trade name, Nippon Kayaku); epirubicin as Pharmorubicin (trade name, Pharmacia K.K.); zinostatin stimalamer as SMANCS (trade name, Yamanouchi Pharmaceutical); and idarubicin as Idamicin (trade name, Pharmacia K.K.).

The plant-derived antitumor agents mentioned above are commercially available: for example, vincristine as Oncovin (trade name, Shionogi & Co., Ltd.); vinblastine as Vinblastine (trade name, Kyorin Pharmaceutical); vindeshine as Fildesin (trade name, Shionogi & Co., Ltd.); etoposide as Lastet (trade name, Nippon Kayaku); sobuzoxane as Perazolin (trade name, ZENYAKU KOGYO CO., LTD.); docetaxel as Taxotere (trade name, Aventis); paclitaxel as Taxol (trade name, Bristol); and vinorelbine as Navelbine (trade name, KYOWA HAKKO KOGYO Co., Ltd.).

The antitumor platinum-complex compounds mentioned above are commercially available: for example, cisplatin as Randa (trade name, Nippon Kayaku); carboplatin as Paraplatin (trade name, Bristol); nedaplatin as Aqupla (trade name, Shionogi & Co., Ltd.); and oxaliplatin (Desynth S.A., U.S.A.).

The antitumor campthotecin derivatives mentioned above are commercially available: for example, irinotecan as Campto (trade name, Yakult); topotecan (Qventas, U.S.A.); and campthotecin (Aldrich Chemical Company, Inc, U. S.A.).

The interferons mentioned above are commercially available; for example, interferon $\alpha$ as Sumiferon (trade name, Sumitomo Pharmaceuticals Co., Ltd.); interferon $\alpha$-2a as Canferon-A (trade name, Takeda Chemical Ind.); interferon $\alpha$-2b as Intron A (trade name, Schering-Plough); interferon $\beta$ as IFN$\beta$ (trade name, MOCHIDA PHARMACEUTICAL CO., LTD.); interferon $\gamma$-1a as Imunomax-$\gamma$ (trade name, Shionogi & Co., Ltd.); and interferon $\gamma$-n1 as Ogamma (trade name, Otsuka Pharmaceutical Co., Ltd.).

The biological response modifiers mentioned above are commercially available: for example, krestin as Krestin (trade name, Sankyo Co); lentinan as Lentinan (trade name, Aventis); sizofiran as Sonifiran (trade name, Kaken Pharmaceutical Co., Ltd.); picibanil as Picibanil (trade name, Chugai Pharmaceutical Co., Ltd.); and ubenimex as Bestatin (trade name, Nippon Kayaku).

The other antitumor agents mentioned above are commercially available: for example, mitoxantrone as Novantrone (trade name, Wyeth Lederle (Japan) Ltd.); L-asparaginase as Leunase (trade name, KYOWA HAKKO KOGYO Co., Ltd.); procarbazine as Natulan (trade name, Nippon Roche K.K.); dacarbazine as Dacarbazine (trade name, KYOWA HAKKO KOGYO Co., Ltd.); hydroxycarbamide as Hydrea (trade name, Bristol); pentostatin as Coforin (trade name, Kagaku oyobi Kessei Ryouhou Kenkyuusho); and tretinoin as Vesanoid (Nippon Roche K.K.).

In the method according to the present invention, a preferred therapeutic unit may vary in accordance with: for example, a medication form of the compound of general formula I, a type of the compound of general formula I to be used, and a dosage form of the compound of general formula I to be used; a type of other antitumor agent(s) to be used together with, a medication form and a dosage form thereof; and a type of cancer cells and condition of the patient to be treated. Those skilled in the art can determine an optimal treatment under a given condition based on the set conventional therapeutic unit and/or by taking the disclosure of the present specification into consideration.

More specifically, the therapeutic unit of the compound of general formula I in the method according to the present invention can vary in accordance with, for example, a type of the compound to be used, a type of the composition incorporated, the application frequency and a specific site to be treated, a level of disease, the age of the patient, the doctor's diagnosis, and a type of cancer. As a tentative measure, for example, the dose for an adult per day can be in

the range of 1 to 500 mg in the case of oral administration. In the case of parenteral administration, preferably intravenous administration, and more preferably intravenous drip infusion, for example, the dose per day can be in the range of 1 to 100 $mg/m^2$ (body surface area), preferably 10 to 15 $mg/m^2$ (body surface area), and more preferably 13 $mg/m^2$ (body surface area). In the case of intravenous drip infusion, for example, administration may be continuously carried out for 1 to 4 hours, preferably 2 to 3 hours, and more preferably for 2 hours. The administration frequency varies in accordance with the medication methods and symptoms; for example, 1 to 5 times, preferably 1 or 2 times, and more preferably once a day. Alternatively, periodical intermittent medication, such as medication every other day or every two days, may be employed. The period of withdrawal from medication in the case of parenteral administration is, for example, for 1 to 6 weeks, preferably for 2 to 4 weeks, and more preferably for 3 to 4 weeks.

The therapeutic unit of the other antitumor agent to be used together with the compound of general formula I is not particularly limited to, but it can be optionally determined by those skilled in the art in accordance with the known literature. Examples are given in the following:

The therapeutic unit of 5-fluorouracil (5-FU) is as follows: in the case of oral administration, for example, 200 to 300 mg is daily administered 1 to 3 times per day; in the case of injections, for example, 5 to 15 mg/kg per day administered by once-a-day intravenous injection or intravenous drip infusion for the first consecutive 5 days, followed by the administration of 5 to 7.5 mg/kg by once-a-day intravenous injection or intravenous drip infusion every other day (wherein the dose may be suitably decreased or increased).

The therapeutic unit of S-1 (Tegafur · Gimestat · Otastat potassium) is that, for example, the initial dose (single dose) is set at the following standard amount in accordance with the body surface area; and it is daily administered orally twice a day, i.e., after breakfast and after dinner, for consecutive 28 days, followed by withdrawal from medication for 14 days. This is set as one course, and administration of such course is repeated. The initial standard amount per body surface area (Tegaful equivalent) is less than 1.25 $m^2$: 40 mg/dose, 1.25 $m^2$ or more to less than 1.5 $m^2$: 50 mg/dose, 1.5 $m^2$ or more: 60 mg/dose. It is suitably decreased or increased in accordance with the patient's conditions.

The therapeutic unit of gemcitabine is that, for example, 1 $g/m^2$ of gemcitabine is administered per dose by intravenous drip infusion for 30 minutes, and once-weekly administration per week is continued for 3 weeks, followed by withdrawal from medication on the 4th week. This is set as one course, and administration of such course is repeated. The dose is suitably decreased in accordance with the age, symptom, or the development of side effects.

The therapeutic unit of doxorubicin (e.g., doxorubicin hydrochloride) is as follows: in the case of intravenous injection, for example, 10 mg (0.2 mg/kg) (potency) is one-shot administered intravenously once a day for consecutive 4 to 6 days, followed by withdrawal from medication for 7 to 10 days. This procedure is set as one course and repeated 2 or 3 times. The total dose is preferably not more than 500 mg (potency)/$m^2$ (body surface area), and it may be suitably decreased or increased within this range.

The therapeutic unit of etoposide is as follows: in the case of intravenous injection, for example, 60 to 100 $mg/m^2$ (body surface area) per day is continuously administered for 5 days, followed by withdrawal from medication for 3 weeks (wherein the dose may be suitably decreased or increased). This procedure is repeated as one course. In contrast, in the case of oral administration, for example, 175 to 200 mg per day is continuously administered for 5 days, followed by withdrawal from medication for 3 weeks (wherein the dose may be suitably decreased or increased). This procedure is repeated as one course.

The therapeutic unit of docetaxel (docetaxel hydrate) is that, for example, 60 $mg/m^2$ (body surface are) of docetaxel is administered over the period of 1 hour or longer once a day, and the administration is carried out by intravenous drip infusion at the interval of 3 to 4 weeks (wherein the dose may be suitably decreased or increased).

The therapeutic unit of paclitaxel is that, for example, 210 $mg/m^2$ (body surface are) is administered over the period of 3 hours once a day by intravenous drip infusion, followed by withdrawal from medication for at least 3 weeks. This procedure is repeated as one course. The dose may be suitably decreased or increased.

The therapeutic unit of cisplatin is as follows: in the case of intravenous injection, for example, 50 to 70 $mg/m^2$ (body surface are) is administered once a day, followed by withdrawal from medication for 3 weeks or longer (wherein the dose may be suitably decreased or increased). This procedure is repeated as one course.

The therapeutic unit of carboplatin is that, for example, 300 to 400 $mg/m^2$ is administered once a day by intravenous drip infusion for 30 or more minutes, followed by withdrawal from medication for at least 4 weeks (wherein the dose may be suitably decreased or increased). This procedure is repeated as one course.

The therapeutic unit of oxaliplatin is that 85 $mg/m^2$ is administered by intravenous injection once a day, followed by withdrawal from medication for 2 weeks. This procedure is repeated as one course.

The therapeutic unit of irinotecan (e.g., irinotecan hydrochloride) is that, for example, 100 $mg/m^2$ once a day is administered by intravenous drip infusion 3 to 4 times at the interval of 1 week, followed by withdrawal from medication for at least 2 weeks.

The therapeutic unit of topotecan is that, for example, 1.5 $mg/m^2$ is administered by intravenous drip infusion once a day for 5 days, followed by withdrawal from medication for at least 3 weeks.

The therapeutic unit of cyclophosphamide is as follows. In the case of intravenous injection, for example, 100

mg once a day is administered by intravenous injection every day. When the patient can cope with it, it may be increased to 200 mg per day. In total, 3,000 to 8,000 mg is administered; however, it may be suitably decreased or increased. If necessary, it may be injected or infused intramuscularly, intrathoracically, or intratumorally. In contrast, in the case of oral administration, for example, 100 to 200 mg is administered per day.

The therapeutic unit of Iressa is that, for example, oral administration of 250 mg once a day.

The therapeutic unit of SU5416 is, for example, 145 mg/m$^2$ of SU541 is administered by intravenous drip infusion over the period of 60 minutes twice a week, and this administration is continued for 4 weeks. This procedure is repeated as one course.

The therapeutic unit of IMC-C225 is that, for example, 400 mg/m$^2$ of IMC-C225 is administered by intravenous drip infusion on the first day, followed by intravenous drip infusion of 250 mg/m$^2$ thereof every week.

The therapeutic unit of RhuMabVEGF is, for example, intravenous drip infusion of 3 mg/kg every week.

When 5-FU is combined with leucovorin, the therapeutic unit is that, for example, 425 mg/m$^2$ of 5-FU and 200 mg/m$^2$ of leucovorin are administered by intravenous drip infusion from the first day to the fifth day. This is repeated at the interval of 4 weeks.

Each preparation that is contained in the combined preparation of the present invention can be various forms, and examples thereof include: oral preparations such as tablets, capsules, powders, granules, or solvents; and sterilized liquid parenteral preparations, suppositories, or ointments such as solutions or suspensions.

Solid preparations can be produced as it is in the form of tablets, capsules, powders, or granules, and can also be produced using suitable carriers (additives). Examples of such carriers (additives) include: saccharides such as lactose and glucose; starch such as corn, wheat, and rice; fatty acids such as stearic acid; inorganic salts such as magnesium aluminometasilicate and anhydrous calcium phosphate; synthetic polymers such as polyvinyl pyrrolidone and polyalkylene glycol; fatty acid salts such as calcium stearate and magnesium stearate; alcohols such as stearyl alcohol and benzyl alcohol; synthetic cellulose derivatives such as methyl cellulose, carboxymethyl cellulose, ethyl cellulose, or hydroxypropylmethyl cellulose; and commonly used additives such as gelatin, talc, vegetable oil, or gum arabic.

In general, these solid preparations such as tablets, capsules, granules, and powders may comprise, on the basis of the weight of the entire preparation, for example, 0.1 to 100% by weight, preferably 5 to 100% by weight, more preferably 5 to 60% by weight, of the compound of general formula I, and particularly preferably 5 to 40% by weight of the active ingredient.

Liquid preparations are produced in the form of a suspension, syrup, injection, infusion (intravenous blood transfusion), and the like by using water, an alcohol, or a suitable additive which is commonly used in liquid preparations such as vegetable-derived oil such as soybean oil, peanut oil, or sesame oil.

Particularly, when the preparation is administered parenterally by intramuscular injection, intravenous injection, or subcutaneous injection, examples of a suitable solvent or diluent include distilled water for injection, an aqueous solution of lidocaine hydrochloride (for intramuscular injection), physiological saline, an aqueous solution of glucose, ethanol, polyethylene glycol, propylene glycol, a liquid for intravenous injection (e.g., an aqueous solution of citric acid and sodium citrate), or an electrolyte solution (for intravenous drip infusion and intravenous injection) or a mixed solution thereof.

These injections can be in the form of solution which was solved in advance or alternatively they can be in the form of powder, or in the form of a suitable carrier (additive) added being dissolved just before administration. These injections can comprise, for example, 0.1 to 10% by weight, and preferably 0.1 to 5% by weight of the active ingredient, based on the weight of the entire preparation.

Liquid preparations such as a suspension or syrup for oral administration can comprise, for example, 0.5 to 10% by weight of the active ingredient based on the weight of the entire preparation.

Each preparation in the combined preparation according to the present invention can also be easily produced by those skilled in the art in accordance with conventional or common techniques. For example, when a preparation (s) comprising other antitumor agent(s) that is(are) used in combination with the compound of general formula I is(are) an oral preparation, the preparation(s) can be produced by, for example, mixing a suitable amount of the antitumor agent with a suitable amount of lactose and filling the mixture into a hard gelatin capsule, which is suitable for oral preparation. In contrast, when a preparation comprising the antitumor agent is an injection, the preparation can be produced by, for example, mixing a suitable amount of the antitumor agent with a suitable amount of 0.9% physiological saline and filling this mixture into a vial for injection.

Also, a combined preparation of the present invention, which comprises the compound of general formula I and other antitumor agent(s), can be easily produced by those skilled in the art in accordance with conventional or common techniques.

Brief Description of the Drawings

Fig. 1 shows a synergistic effect exhibited by the combined use of compound A and cisplatin. Fig. 2 shows a synergistic effect exhibited by the combined use of compound A and carboplatin. Fig. 3 shows a significant inhibition of tumor growth by the effect exhibited by the combined use of compound A and 5-FU/leucovorin.

Best Mode for Carrying out the Invention

The present invention will be described in more detail with reference to the following examples, although the present invention is not limited to these examples only.

Production examples for a preparation comprising the compound of general formula I in the combined preparation according to the present invention are first described. Preparations other than the preparation comprising the compound of general formula I in the combined preparation according to the present invention are commercially available, or they can be easily produced by those skilled in the art in accordance with conventional literature or common techniques.

Preparation Example 1

Production of a preparation for injection comprising the compound of formula IA (compound A):

Distilled water for injection (55 L) is heated at 60 to 65°C, and 6 kg of glucose (the Pharmacopoea of Japan) is added and stirred to dissolve therein. A suitable amount of the heated distilled water for injection is added to bring the amount to 60 L; 18 g of compound A is added thereto; and the mixture is stirred and dissolved within 2 hours. The cooled distilled water for injection (58 L) is added and mixed therein by stirring. After the mixture is cooled to room temperature, distilled water for injection is added to bring the amount to 120 L. After the obtained solution is disinfected and filtrated through a 0.22 μ filter, the filtrate is filled and hermetically sealed in a sterilized plastic bag in an amount of 255 to 265 mL per bag.

Preparation Example 2

Production of an oral preparation comprising the compound of formula IA (compound A):

4,500 g of Lactose, 3,950 g of crystalline cellulose, 200 g of hydroxypropyl cellulose, 300 g of crosscarmellose sodium, and 1,000 g of compound A are stirred and mixed in a high-speed stirring granulator. 2,000 g of purified water is added and stirred for granulation. After the granules were dried in a fluidized bed dryer,. they are pulverized by Comill. The obtained granules and 50 g of magnesium stearate are mixed in a V-shape mixer for lubrication. The product is then subjected to compression molding in a tablet-making machine to prepare into 200 mg tablets.

Example 1

Effect of a combined use of drugs using cells

a) Reagent

Fetal calf serum (FCS) was obtained from Moregate, and DMEM medium was obtained from Asahi Techno Glass Corporation. Compound A (the compound of formula IA) was synthesized in accordance with the method by Ohkubo et al. (M. Ohkubo et al., Bioorg. & Med. Chem. Lett., 9, 3307-3312 (1999)). Cisplatin (CDDP, randa injection) was obtained from Nippon Kayaku; campthotecin (CPT) was obtained from Sigma; adriamycin (ADM, Adriacin) was obtained from KYOWA HAKKO KOGYO Co., Ltd.; vincristine (VCR, Oncovin) was obtained from Shionogi & Co. Ltd.; and carboplatin (CBDCA) was obtained from Sigma.

b) Cells

HCT116 human large intestine cancer cells, SCC-25 human tongue cancer cells, A427 human non-small cell lung cancer cells, and J82 human bladder cancer cells were obtained from American Type Culture Collection (ATCC).

c) Evaluation method of effect

Cells were suspended in 10% FCS-added DMEM medium, and a cell suspension in an amount of 1,000 cells/50 µl/well was inoculated on a 96-well plastic plate. Culture was conducted at 37°C in 5% $CO_2$-95% air overnight. Each drug was diluted with dimethyl sulfoxide or a suitable solvent, and 50 µl each of one drug or two drugs were added on the plate having cells inoculated thereon. Culture is further conducted for 3 days at 37°C in 5% $CO_2$-95% air. Cell growth was mesured by the WST-8 method (H. Tominaga et al., Anal. Commun., 36, 47-50 (1999)). The WST-8 method used herein refers to a method comprising the steps of adding 10 µl of WST-8 reagent solution to each well, continuing the culture for 1 to 6 hours at 37°C, stirring the plate, measuring the amount of formazan produced by colorimetry, and determining the inhibitory rate of the drug.

The drug interaction of two drugs was evaluated by the isobologram method (G. G. Steel and M. Peckham, Int. J. Radiat. Oncol. Biol. Phys., 5, 85-91 (1979)). In accordance with the isobologram method, the 50% growth inhibitory concentration ($IC_{50}$) of each drug was determined, and graphs were prepared using cell survival ratios and relative concentrations (the concentration of the drug when $IC_{50}$ is 1.0). Subsequently, the graph obtained from each drug was used; and the case where the drugs, which were used together, were a combination of drugs having action mechanisms different from each other (mode 1) and the case where the drugs had similar action mechanisms (mode 2) were hypothesized; and two lines were plotted (see Fig. 1). When the point representing the cell growth inhibitory ratio in the case of actual combined use was present in the region sandwiched between these two lines, the drug interaction was evaluated as an additive effect. When the point was below the region surrounded by the two lines, it was evaluated as an synergistic effect. When the point was above the region, it was evaluated as an antagonistic effect. Fig. 1 shows a synergistic effect exhibited by the combined use of compound A and cisplatin. Fig. 2 shows a synergistic effect exhibited by the combined use of compound A and carboplatin. The evaluation of effects is described in the following.

Table 1:

| The effect of a combined use of compound A and other antitumor agent | | | | |
|---|---|---|---|---|
| Type of cell | CDDP | CPT | ADM | CBDCA |
| HCT116 | S | S | - | S |
| SCC-25 | S | S | - | - |
| A427 | S | S | - | - |
| J82 | S | S | S | - |
| S: synergistic effct, CDDP: cisplatin, CPT: campthotecin, ADM: adriamycin (doxorubicin), CBDCA: carboplatin | | | | |

As is apparent from Table 1, compound A exhibits synergistic antitumor effects by the combined use with cisplatin, campthotecin, adriamycin, or carboplatin.

Example 2

Effect of a combined use of drugs using animals (1)

a) Mouse and cancer cells

Female CDF1 mice (4- or 5-week old) were obtained from Charles River Japan, Inc. P388 mouse leukemia cells were obtained from the National Cancer Center Institute.

b) Reagent

Compound A, cisplatin, etoposide, and adriamycin (doxorubicin) are the same as in Example 1.

c) Evaluation method of effect

P388 mouse leukemia cells (1 x $10^6$ cells) were transplanted intraperitoneally per mouse (day 0). One drug or two drugs were intraperitoneally administered on the next day (day 1). The control group which was not treated with drugs and the group which was treated with drugs were observed for the number of days they survived. The mice who had survived by the end of the experiment (day 30) and did not retain peritoneal fluids were determined as complete remission, and calculation was made by determining the number of days they survived as 60 days. The ratio of increased life span (ILS%) was determined according to the following equation:

ILS% = ((the number of survived days for the group treated with drug) / (the number of survived days for the control group without drug) - 1) x 100

The combination index (CI) showing combination effect was determined by the following equation in accordance with the method by Uchida et al. (Uchida et al., Gan to Kagaku Ryouhou (Cancer and Chemotherapy), 25, 75-78 (1998)) based on the ILS% obtained from the treatment using one drug and the ILS% obtained from the combined treatment.

CI = ILS% of the combined treatment / (ILS% with J-107088 (Compound A) alone + ILS% with another drug)

The effect exhibited by the combined use of two drugs was evaluated as follows based on CI.

CI > 1: synergistic effect, CI = 1: additive effect, CI < 1: antagonistic effect

Table 2:

| Effect of combined use using compound A and other antitumor agent | | | | | | |
|---|---|---|---|---|---|---|
| Drug 1 | Dose (mg/kg) | Drug 2 | Dose (mg/kg) | Number of days survived (average ± standard deviation) | Increased life span (ILS%) | CI |
| Control | - | - | - | 11.5 ± 1.0 | 0 | - |
| Compound A | 75 | - | - | 18.0 ± 1.2 | 57 | - |
| - | - | CDDP | 1.67 | 25.4 ± 19.6 | 121 | - |
| - | - | ADM | 1.5 | 19.0 ± 1.9 | 65 | - |
| - | - | VP-16 | 7.5 | 17.4 ± 4.1 | 51 | - |
| Compound A | 75 | CDDP | 1.67 | 39.6 ± 18.6 | 244 | 1.38 |
| Compound A | 75 | ADM | 1.5 | 35.6 ± 22.3 | 210 | 1.72 |
| Compound A | 75 | VP-16 | 7.5 | 52.8 ± 16.1 | 359 | 3.33 |
| CDDP: cisplatin, ADM: adriamycin (doxorubisin), VP-16: etoposide | | | | | | |

As is apparent from Table 2, compound A exhibits synergistic antitumor effects by the combined use with cisplatin, adriamycin, or etoposide.

Example 3

Effect of combined use of drugs using animals (2)

a) Mouse and cancer cells

Female nude mice (5-week old) were obtained from Charles River Japan, Inc. Human large intestine cancer HCT116 cells were obtained from American Type Culture Collection.

b) Reagent

Compound A was synthesized in accordance with the method by Ohkubo et al. as in Example 1. 5-Fluorouracil (5-FU) was obtained from KYOWA HAKKO KOGYO Co., Ltd., and leucovorin (trade name, Isovorin injection) was obtained from Takeda Chemical Ind.

c) Evaluation method of effect

The HCT116 cells, which are continuously maintained subcutaneously in nude mice, were sliced in 3-mm square, and a piece was subcutaneously transplanted to each of nude mice that were to be subjected to the experiment. When the subcutaneous tumor volume reached 0.07 $cm^3$ to 0.36 $cm^3$, mice were randomly divided into 4 groups as shown in Table 3 (day 0).

Table 3:

| Grouping in Example 3 | |
|---|---|
| Group No. | Treatment |
| 1 | Control group (only 0.5% glucose solution and physiological saline were administered) |
| 2 | Group to which 0.3 mg/kg of compound A was administered |
| 3 | Group to which 10 mg/kg of 5-FU + 83.3 mg/kg of leucovorin were administered |
| 4 | Group to which 0.3 mg/kg of compound A + 10 mg/kg of 5-FU + 83.3 mg/kg of leucovorin were administered |

Compound A (0.3 mg/kg) was administered into the tail vein on days 0 and 7. Leucovorin (83.3 mg/kg) was administered into the caudate vein on days 2, 4, 9, and 11. One hour after the leucovorin administration, 10 mg/kg of 5-FU was administered into the tail vein.

The tumor volume was determined by the following equation based on the longer axis and the shorter axis of the tumor:

$$\text{Tumor volume } (cm^3) = (\text{longer axis (cm)} \times (\text{shorter axis (cm)})^2 / 2).$$

The tumor volume was measured with time to compare with the tumor volume when the administration was initiated. This value was subject to the test evaluating significant differences (Mann-Whiteney's U-test) among the group to which only compound A was administered, the group to which 5-FU + leucovorin were administered, and the group to which compound A + 5-FU + leucovorin were administered. The group to which compound A + 5-FU + leucovorin were administered was determined to be significantly different ($P < 0.05$) from the group to which only compound A was administered and the group to which 5-FU + leucovorin were administered; and these drugs were judged to have the effect exhibited by the combined use (see Table 4). Fig. 3 shows a significant inhibition of tumor growth generated by the combined use of compound A and 5-FU/leucovorin.

Table 4:

| Table 4: Tumor volume as of 14 days after the initiation of administration and growth inhibitory effect exhibited by combined treatment | | | |
|---|---|---|---|
| Group No. | Average tumor volume ($cm^3$) | Standard deviation ($cm^3$) | Growth inhibitory rate (%) |
| 1 | 4.52 | 0.41 | 0 |
| 2 | 4.27 | 0.14 | 6 |
| 3 | 4.44 | 0.47 | 2 |
| 4 | 3.17 | 0.12 | 30 |

Example 4

Effect of lowering toxicity by the combined use of drugs

a) Mouse and cancer cells

Female CDF1 mice (4- or 5-week old) and P388 mouse leukemia cells are the same as in Example 2.

b) Reagent

Compound A and adriamycin (doxorubicin) are the same as in Example 1.

c) Evaluation method of effect

P388 mouse leukemia cells (1 x $10^6$ cells) were transplanted intraperitoneally per mouse (day 0). One drug or two drugs were intraperitoneally administered on the next day (day 1). The control group which was not treated with drugs, and the group which was treated with drugs were observed for the number of days they survived. The mice who had survived by the end of the experiment (day 30) and did not retain peritoneal fluids were determined as complete recovery, and calculation was made by regarding the number of days they survived as 60 days. The ratio of increased life span (ILS%) was determined in the same manner as in Example 2.

The dose which exhibits the equivalent ILS% in the use of compound A alone, in the use of ADM alone, or in the combined use of compound A and ADM, was determined. This dose was intraperitoneally administered to mice once a day for consecutive 5 days. Thereafter, whether the animal was alive or dead was observed, and the effect of lowering toxicity was judged. As the toxicity index, the changes in the body weight between that before the administration and that on the subsequent day after the administration were recorded.

Table 5:

| Effect of combined use of compound A and ADM | | | | |
|---|---|---|---|---|
| Drug 1 | Dose (mg/kg) | Drug 2 | Dose (mg/kg) | Ratio of increased life span (ILS%) |
| Control | - | - | - | 0 |
| Compound A | 25 | - | - | 79 |
| - | - | ADM | 1.5 | 92 |
| Compound A | 5 | ADM | 0.3 | 83 |
| ADM: adriamycin (doxorubisin) | | | | |

Table 6:

| Effect of lowering toxicity by the combined use of compound A and ADM | | | | | |
|---|---|---|---|---|---|
| Drug 1 | Dose (mg/kg) | Drug 2 | Dose (mg/kg) | Number of dead animals / number of animals per group | Change in body weight (g) |
| Control | - | - | - | 0/4 | +0.8 |
| Compound A | 25 | - | - | 4/4 | - |
| - | - | ADM | 1.5 | 0/4 | +0.1 |
| Compound A | 5 | ADM | 0.3 | 0/4 | -0.3 |
| ADM: adriamycin (doxorubisin) | | | | | |

As is apparent from Tables 5 and 6, the combined use of compound A with adriamycin can significantly decrease the amount of each drug, and it exhibited the effect of reducing side effects.

Industrial Applicability

The compound of general formula I (particularly compound A) and other antitumor agents (e.g., cisplatin, adriamycin (doxorubicin), carboplatin, and 5-FU/leucovorin) exhibited synergistic antitumor activities *in vitro* or *in vivo*. The combined administration of these drugs significantly decreased the amount of each drug (e.g., the combined use of compound A and adriamycin (doxorubicin)). Accordingly, it can be expected that the use of the compound of general formula I (particularly compound A) with other drugs can be applied for wider applications. Specifically, when the compound of general formula I (particularly compound A) exhibits synergistic effects with other drugs, the dose of the compound of general formula I (particularly compound A) can be smaller than the case when it is administered alone.

This also led to alleviation of side-effects, which is a side benefit.

## Claims

**1.** A combined preparation for simultaneous, separate, or sequential administration in the treatment of cancer, comprising two separate preparations:

* a preparation comprising, in combination with a pharmaceutically acceptable carrier or diluent, at least one compound of general formula I:

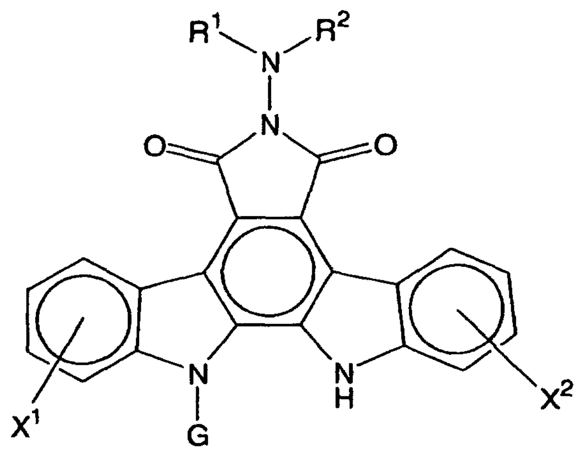

[ I ]

[wherein $R^1$ and $R^2$ each independently represent:

a hydrogen atom, lower alkyl, lower alkenyl, lower alkynyl, aryl, aralkyl, or heterocyclic group (wherein the lower alkyl, the lower alkenyl, the lower alkynyl, the aryl, the aralkyl, and the heterocyclic group may each have one to five of the same or different substituents selected from the group consisting of carboxyl, carbamoyl, sulfo, amino, cyano, mono-lower alkylamino, di-lower alkylamino, hydroxyl, and a halogen atom);
or a group of formula -Y-$R^3$ {wherein Y represents carbonyl, thiocarbonyl, or sulfonyl, and $R^3$ represents a hydrogen atom, lower alkyl, cycloalkyl, cycloalkyl-lower alkyl, aryl, aralkyl, lower alkoxy, hydrazino, amino, arylamino, carbamoyl, or heterocyclic group (wherein the lower alkyl, the cycloalkyl, the cycloalkyl-lower alkyl, the aryl, the aralkyl, and the heterocyclic group may each have one to four of the same or different substituents selected from the group consisting of a halogen atom, optionally protected hydroxyl, amino, carboxyl, carbamoyl, cyano, and lower alkoxycarbonyl in which the amino and the carbamoyl may each be further mono- or di-substituted by lower alkyl optionally substituted by a substituent or substituents selected from the group consisting of a halogen atom, hydroxyl, amino, carboxyl, carbamoyl, and lower alkoxycarbonyl)); or
a group of formula $-(CH_2)_m-R^4$ {wherein $R^4$ is pyridyl, furyl, or thienyl (wherein the pyridyl, the furyl, and the thienyl may each have one or two substituents selected from the group consisting of hydroxyl, lower alkoxy, hydroxy-lower alkyl, and hydroxy-lower alkenyl), and m is an integer of 1 to 3},
$R^1$ and $R^2$ are combined together to represent lower alkylidene (wherein the lower alkylidene may have one to four of the same or different substituents selected from the group consisting of amino, mono-lower alkylamino, di-lower alkylamino, hydroxyl, carboxyl, and sulfo), or
$R^1$ and $R^2$, together with the nitrogen atom to which they bind, form heterocyclic group (wherein the heterocyclic group may have, on said ring, lower alkyl optionally substituted by a group or groups selected from the group consisting of amino, hydroxyl, carboxyl, and sulfo),
G represents a pentosyl or hexosyl; and
$X^1$ and $X^2$ each independently represent a hydrogen atom, a halogen atom, amino, mono-lower alkylamino, di-lower alkylamino, hydroxyl, lower alkoxy, aralkoxy, carboxyl, lower alkoxycarbonyl, or lower alkyl]
or a pharmaceutically acceptable salt thereof; and

* a preparation comprising, in combination with a pharmaceutically acceptable carrier or diluent, at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents or a pharmaceutically acceptable salt thereof

(wherein the antitumor alkylating agents are nitrogen mustard N-oxide, cyclophosphamide, ifosfamide, melphalan, busulfan, mitobronitol, carboquone, thiotepa, ranimustine, nimustine, or temozolomide,
the antitumor antimetabolites are methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, or fludarabine,
the antitumor antibiotics are actinomycin D, doxorubicin, daunorubicin, neocarzinostatin, bleomycin, peplomycin, mitomycin C, aclarubicin, pirarubicin, epirubicin, zinostatin stimalamer, or idarubicin,
the plant-derived antitumor agents are vincristine, vinblastine, vindeshine, etoposide, sobuzoxane, docetaxel, paclitaxel, or vinorelbine,
the antitumor platinum-complex compounds are cisplatin, carboplatin, nedaplatin, or oxaliplatin,
the antitumor campthotecin derivatives are irinotecan, topotecan, or campthotecin,
the antitumor tyrosine kinase inhibitors are Iressa or SU5416,
the monoclonal antibodies are IMC-C225, RhuMabVEGF, or Rituximab,
the interferons are interferon $\alpha$, interferon $\alpha$-2a, interferon $\alpha$-2b, interferon $\beta$, interferon $\gamma$-1a, or interferon $\gamma$-n1,
the biological response modifiers are krestin, lentinan, sizofiran, picibanil, or ubenimex, and
the other antitumor agents are mitoxantrone, L-asparaginase, procarbazine, dacarbazine, hydroxycarbamide, pentostatin, or tretinoin).

**2.** A combined preparation for simultaneous, separate, or sequential administration in the treatment of cancer, comprising two separate preparations:

* a preparation comprising, in combination with a pharmaceutically acceptable carrier or diluent, at least one compound of general formula I as defined in Claim 1 (wherein $R^1$, $R^2$, $R^3$, $R^4$, m, Y, G, $X^1$, and $X^2$ are the same as defined in Claim 1) or a pharmaceutically acceptable salt thereof; and
* a preparation comprising, in combination with a pharmaceutically acceptable carrier or diluent, at least one antitumor agent selected from the group consisting of: 5-fluorouracil; S-1; gemcitabine; doxorubicin and etoposide; docetaxel and paclitaxel; cisplatin, carboplatin, and oxaliplatin; irinotecan, topotecan, and campthotecin; Iressa and SU5416; and IMC-C225 and RhuMabVEGF or a pharmaceutically acceptable salt thereof (wherein, if said preparation contains 5-fluorouracil, it may further contain leucovorin or may be combined with a separate leucovorin preparation).

**3.** A combined preparation as defined in Claim 2, wherein G is a group of formula:

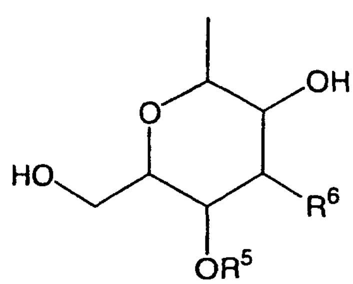

or

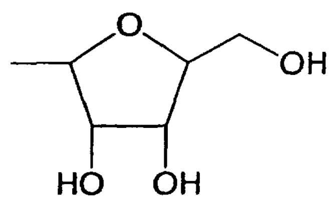

wherein $R^5$ represents a hydrogen atom or lower alkyl, and $R^6$ represents hydroxyl or amino.

**4.** A combined preparation as claimed in Claim 3, wherein $X^1$ and $X^2$ bind to the indolopyrrolocarbazole ring at the 1- or 2-position and at the 10- or 11-position, respectively, and each independently represent a halogen atom, hydroxyl, lower alkoxy, or aralkoxy.

**5.** A combined preparation as claimed in Claim 4, wherein G is β-D-glucopyranosyl, and $X^1$ and $X^2$ represent hydroxyl bonded to the indolopyrrolocarbazole ring at the 2-position and at the 10-position, respectively.

**6.** A combined preparation as claimed in Claim 5, wherein $R^1$ represents a hydrogen atom, and $R^2$ represents a group of formula:

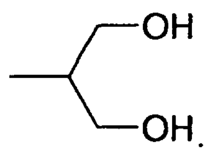

**7.** A combined preparation as claimed in Claim 5, wherein $R^1$ represents a hydrogen atom, and $R^2$ represents $-CH_2-R^4$ in which $R^4$ represents 6-hydroxymethylpyridin-2-yl.

**8.** A combined preparation as claimed in Claim 5, wherein $R^1$ represents a hydrogen atom, and $R^2$ represents $-CH_2-R^4$ in which $R^4$ represents pyridin-4-yl.

**9.** A combined preparation as claimed in Claim 5, wherein $R^1$ represents a hydrogen atom, and $R^2$ represents $-CH_2-R^4$ in which $R^4$ represents 5-hydroxymethylpyridin-4-yl.

**10.** A combined preparation as claimed in Claim 1 or 2, wherein the compound of general formula I as defined in Claim 1 is the compound of formula IA:

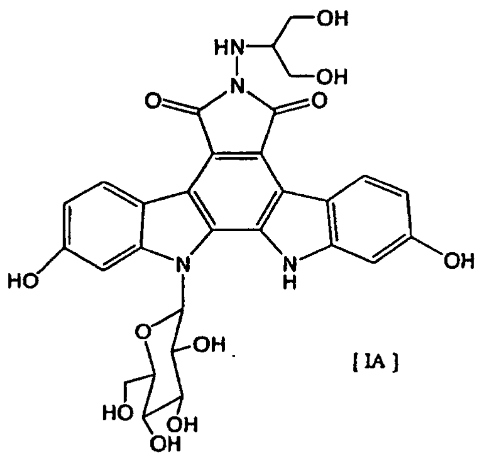

[IA]

**11.** A combined preparation as claimed in Claim 10, wherein one of or both of the two separate preparations according to Claim 1 is/are parenteral preparation(s).

**12.** A combined preparation as claimed in Claim 11, wherein one of or both of the two separate preparations according to Claim 1 is/are an injection or an infusion.

**13.** A combined preparation as claimed in Claim 12, which is further combined with at least one preparation comprising, in combination with a pharmaceutically acceptable carrier or diluent, at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents (wherein a definition of each antitumor agent is the same as defined in Claim 1) or a pharmaceutically acceptable salt thereof.

**14.** A method for cancer treatment, comprising administering to a cancer patient:

(a) a therapeutically effective amount of at least one compound of general formula I as defined in Claim 1 (wherein $R^1$, $R^2$, $R^3$, $R^4$, m, Y, G, $X^1$, and $X^2$ are the same as defined in Claim 1) or a pharmaceutically acceptable salt thereof; in combination with
(b) a therapeutically effective amount of at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents (wherein a definition of each antitumor agent is the same as defined in Claim 1) or a pharmaceutically acceptable salt thereof.

**15.** A method for cancer treatment, comprising administering to a cancer patient:

(a) a therapeutically effective amount of at least one compound of general formula I defined in Claim 1 (wherein $R^1$, $R^2$, $R^3$, $R^4$, m, Y, G, $X^1$, and $X^2$ are the same as defined in Claim 1) or a pharmaceutically acceptable salt thereof; in combination with
(b) a therapeutically effective amount of at least one antitumor agent selected from the group consisting of: 5-fluorouracil; S-1; gemcitabine; doxorubicin and etoposide; docetaxel and paclitaxel; cisplatin, carboplatin, and oxaliplatin; irinotecan, topotecan, and campthotecin; Iressa and SU5416; and IMC-C225 and RhuMab-VEGF or a pharmaceutically acceptable salt thereof (wherein, if the compound of general formula I as defined in Claim 1 is combined with 5-fluorouracil, leucovorin may be further combined).

**16.** A method as claimed in Claim 15, wherein G is a group of formula:

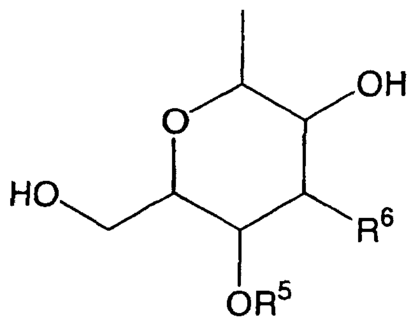

or

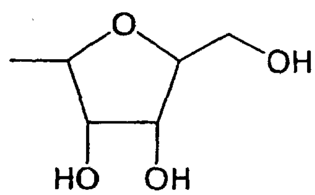

wherein $R^5$ represents a hydrogen atom or lower alkyl, and $R^6$ represents hydroxyl or amino.

**17.** A method as claimed in Claim 16, wherein $X^1$ and $X^2$ bind to the indolopyrrolocarbazole ring at the 1- or 2-position and at the 10- or 11-position, respectively, and each independently represent a halogen atom, hydroxyl, lower alkoxy, or aralkoxy.

**18.** A method as claimed in Claim 17, wherein G is β-D-glucopyranosyl, and $X^1$ and $X^2$ represent hydroxyl bonded to the indolopyrrolocarbazole ring at the 2-position and at the 10-position, respectively.

**19.** A method as claimed in Claim 18, wherein $R^1$ represents a hydrogen atom, and $R^2$ represents a group of formula:

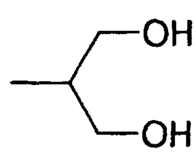

**20.** A method as claimed in Claim 18, wherein $R^1$ represents a hydrogen atom, and $R^2$ represents - $CH_2$-$R^4$ in which $R^4$ represents 6-hydroxymethylpyridin-2-yl.

**21.** A method as claimed in Claim 18, wherein $R^1$ represents a hydrogen atom, and $R^2$ represents -$CH_2$-$R^4$ in which $R^4$ represents pyridin-4-yl.

**22.** A method as claimed in Claim 18, wherein $R^1$ represents a hydrogen atom, and $R^2$ represents - $CH_2$-$R^4$ in which $R^4$ represents 5-hydroxymethylpyridin-4-yl.

**23.** A method as claimed in Claim 14 or 15, wherein the compound of general formula I as defined in Claim 1 is the compound of formula IA:

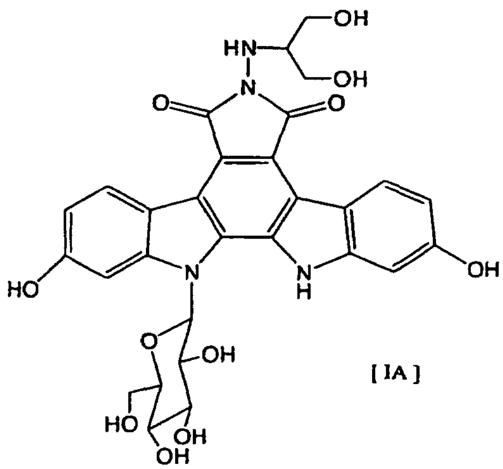

[IA]

**24.** Use of at least one compound of general formula I as defined in Claim I (wherein $R^1$, $R^2$, $R^3$, $R^4$, m, Y, G, $X^1$, and $X^2$ are the same as defined in Claim 1) or a pharmaceutically acceptable salt thereof; and

at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents (wherein a definition of each antitumor agent is the same as defined in Claim 1) or a pharmaceutically acceptable salt thereof, for manufacturing a preparation for cancer treatment.

**25.** Use of at least one compound of general formula I as defined in Claim 1 (wherein $R^1$, $R^2$, $R^3$, $R^4$, m, Y, G, $X^1$, and $X^2$ are the same as defined in Claim 1) or a pharmaceutically acceptable salt thereof; and

at least one antitumor agent selected from the group consisting of 5-fluorouracil; S-1; gemcitabine; doxorubicin and etoposide; docetaxel and paclitaxel; cisplatin, carboplatin, and oxaliplatin; irinotecan, topotecan, and campthotecin; Iressa and SU5416; IMC-C225 and RhuMabVEGF or a pharmaceutically acceptable salt thereof, for manufacturing a preparation for cancer treatment.

**26.** Use as claimed in Claim 25, wherein G is a group of formula:

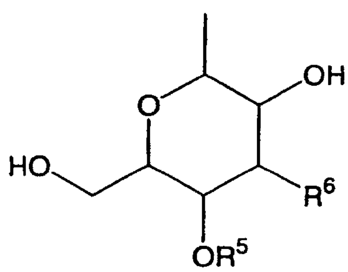

or

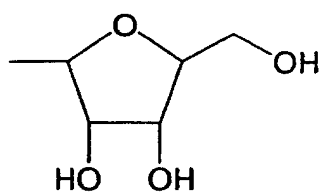

wherein $R^5$ represents a hydrogen atom or lower alkyl, and $R^6$ represents hydroxyl or amino.

**27.** Use as claimed in Claim 26, wherein $X^1$ and $X^2$ bind to the indolopyrrolocarbazole ring at the 1-or 2-position and at the 10- or 11-position, respectively, and each independently represent a halogen atom, hydroxyl, lower alkoxy, or aralkoxy.

**28.** Use as claimed in Claim 27, wherein G is β-D-glucopyranosyl, and $X^1$ and $X^2$ represent hydroxyl bonded to the indolopyrrolocarbazole ring at the 2-position and at the 10-position, respectively.

**29.** Use as claimed in Claim 28, wherein $R^1$ represents a hydrogen atom, and $R^2$ represents a group of formula:

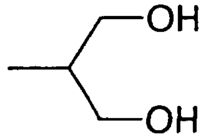

**30.** Use as claimed in Claim 28, wherein $R^1$ represents a hydrogen atom, and $R^2$ represents $-CH_2-R^4$ in which $R^4$ represents 6-hydroxymethylpyridin-2-yl.

**31.** Use as claimed in Claim 28, wherein $R^1$ represents a hydrogen atom, and $R^2$ represents $-CH_2-R^4$ in which $R^4$ represents pyridin-4-yl.

**32.** Use as claimed in Claim 28, wherein $R^1$ represents a hydrogen atom, and $R^2$ represents $-CH_2-R^4$ in which $R^4$ represents 5-hydroxymethylpyridin-4-yl.

**33.** Use as claimed in Claim 24 or 25, wherein the compound of general formula I as defined in Claim 1 is the compound of formula IA:

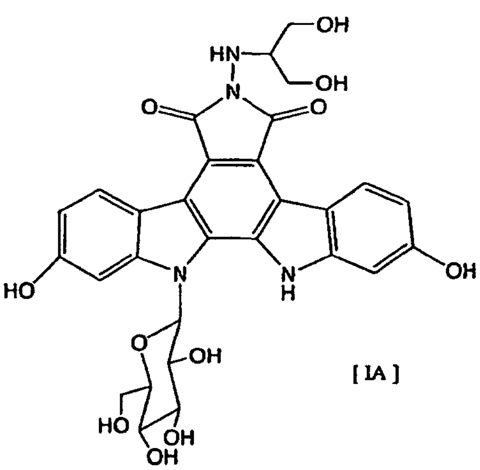

[ IA ]

**34.** A pharmaceutical composition comprising, in combination with a pharmaceutically acceptable carrier or diluent, at least one compound of general formula I as defined above (wherein $R^1$, $R^2$, $R^3$, $R^4$, m, Y, G, $X^1$, and $X^2$ are the same as defined above) or a pharmaceutically acceptable salt thereof; and at least one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, biological response modifiers, and other antitumor agents (wherein a definition of each antitumor agent is the same as defined above) or a pharmaceutically acceptable salt thereof.

**35.** A pharmaceutical composition comprising, in combination with a pharmaceutically acceptable carrier or diluent, at least one compound of general formula I as defined above (wherein $R^1$, $R^2$, $R^3$, $R^4$, m, Y, G, $X^1$, and $X^2$ are the

same as defined above) or a pharmaceutically acceptable salt thereof; and at least one antitumor agent selected from the group consisting of 5-fluorouracil; S-1; gemcitabine hydrochloride; doxorubicin hydrochloride and etoposide; docetaxel hydrate and paclitaxel; cisplatin, carboplatin, and oxaloplatin; irinotecan, topotecan, and campthotecin; Iressa and SU5416; IMC-C225 and RhuMabVEGF or a pharmaceutically acceptable salt thereof (wherein, if said composition contains the compound of general formula I and 5-fluorouracil, it may further contain leucovorin).

FIG. 1

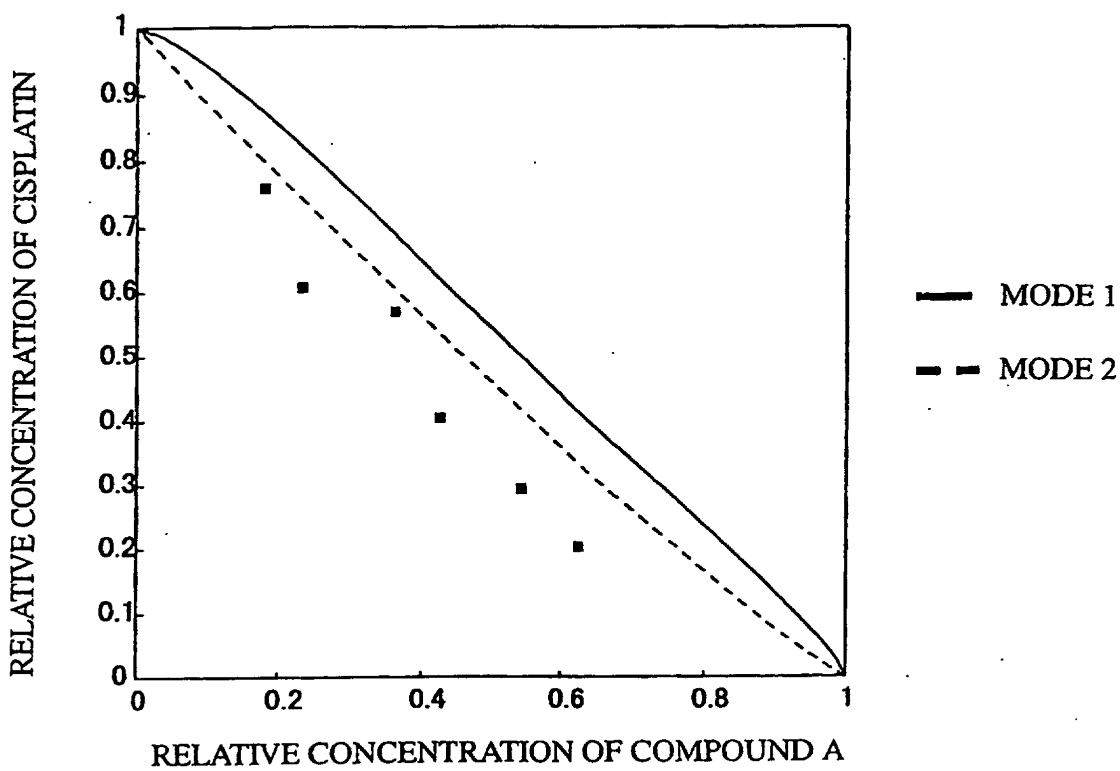
RELATIVE CONCENTRATION OF CISPLATIN
1
0.9
0.8
0.7
0.6
0.5
0.4
0.3
0.2
0.1
0
0
0.2
0.4
0.6
0.8
1
RELATIVE CONCENTRATION OF COMPOUND A
MODE 1
MODE 2

FIG. 2

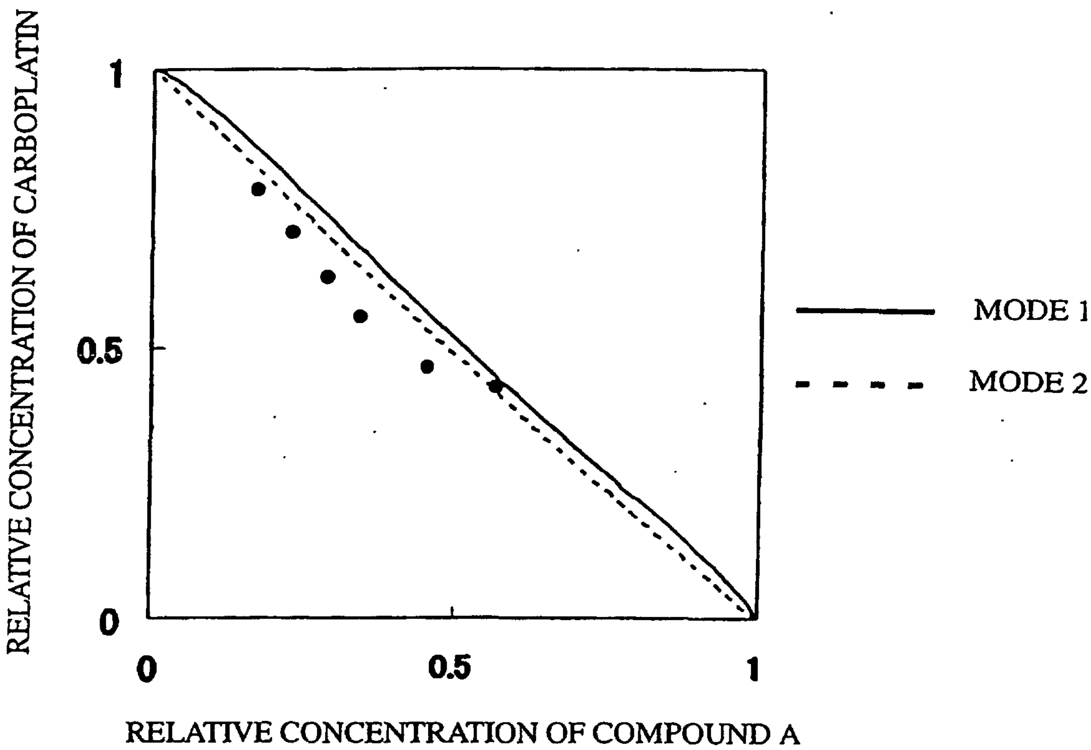
RELATIVE CONCENTRATION OF CARBOPLATIN
1
0.5
0
0
0.5
1
RELATIVE CONCENTRATION OF COMPOUND A
MODE 1
MODE 2

FIG. 3

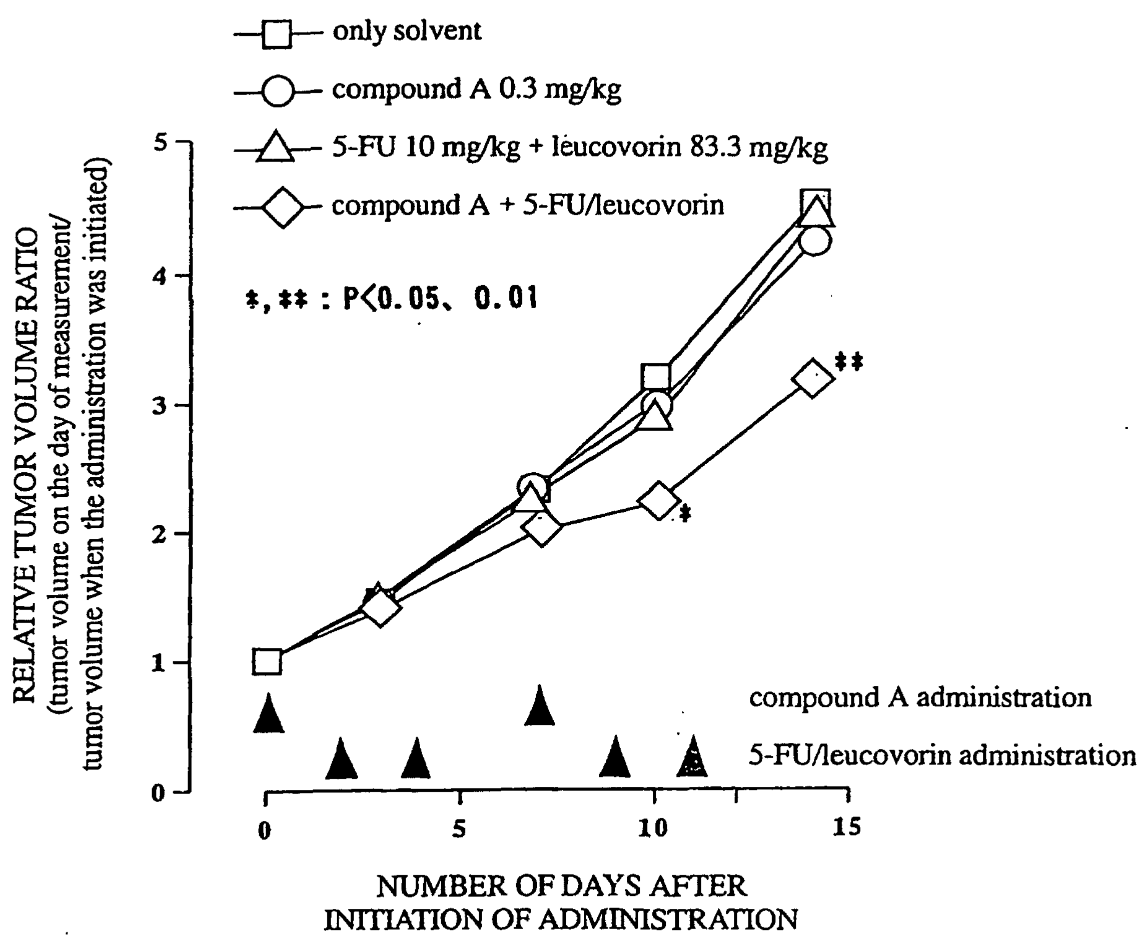
only solvent
compound A 0.3 mg/kg
5-FU 10 mg/kg + leucovorin 83.3 mg/kg
compound A + 5-FU/leucovorin
*, ** : P<0.05、 0.01
RELATIVE TUMOR VOLUME RATIO
(tumor volume on the day of measurement/
tumor volume when the administration was initiated)
0
1
2
3
4
5
0
5
10
15
compound A administration
5-FU/leucovorin administration
NUMBER OF DAYS AFTER
INITIATION OF ADMINISTRATION

## INTERNATIONAL SEARCH REPORT

International application No.
PCT/JP02/10186

**A. CLASSIFICATION OF SUBJECT MATTER**

Int.Cl[7] A61K31/7056, 31/4745, 31/282, 31/24, 31/704, 31/7048, 31/519, 45/00, A61P35/00, 43/00

According to International Patent Classification (IPC) or to both national classification and IPC

**B. FIELDS SEARCHED**

Minimum documentation searched (classification system followed by classification symbols)

Int.Cl[7] A61K31/7056, 31/4745, 31/282, 31/24, 31/704, 31/7048, 31/519, 45/00, A61P35/00, 43/00

Documentation searched other than minimum documentation to the extent that such documents are included in the fields searched

Electronic data base consulted during the international search (name of data base and, where practicable, search terms used)

CAPLUS, REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X | Cancer Research, (1996), 56(4), p.789-93 | 1-13,24-35 |
| X | WO 01/87307 A2 (Celgene Corp.), 22 November, 2001 (22.11.01), (Family: none) | 1-13,24-35 |
| X | EP 545195 A1 (Banyu Pharm. Co., Ltd.), 09 June, 1993 (09.06.93), & CA 2083534 A & IL 103844 A & JP 6-128283 A & AU 9229637 A & NO 9204593 A & WO 93/11145 A1 & HU 65699 A & CN 1073948 A & ZA 9209263 A & CN 1075482 A & US 5589365 A | 1-13,24-35 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 November, 2002 (25.11.02) | 10 December, 2002 (10.12.02) |
| Name and mailing address of the ISA/ Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP02/10186

**Box I Observations where certain claims were found unsearchable (Continuation of item 1 of first sheet)**

This international search report has not been established in respect of certain claims under Article 17(2)(a) for the following reasons:

1. [X] Claims Nos.: 14-23
because they relate to subject matter not required to be searched by this Authority, namely:
The invention as set forth in claims 14 to 23 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)